# EUROPEAN PATENT APPLICATION

(11) **EP 4 212 174 A1**
(43) Date of publication of application: **19.07.2023**
(21) Application number: 21866755.8
(22) Date of filing: 07.09.2021
(51) Int. Cl.: A61K 39/395, A61K 45/00, A61P 35/00, A61P 37/02, C07K 16/18, G01N 33/53

(54) **BIOMARKER FOR PREDICTING RESPONSE TO CANCER TREATMENT**

(30) Priority: 08.09.2020 JP 2020150620
(71) Applicant: Saitama Medical University, Iruma-gun, Saitama 350-0495 (JP)
(72) Inventor: KAGAMU, Hiroshi, Iruma-gun, Saitama 350-0495 (JP)
(74) Representative: Uexküll & Stolberg
(86) International application number: PCT/JP2021/032852
(87) International publication number: WO 2022/054796

(57) **Abstract**

The present invention provides a biomarker for predicting response to cancer treatment and a method of using the biomarker. The present invention provides a method wherein the composition of a subpopulation of cells in a sample, said sample being obtained from a subject, is used as an indicator for predicting response to cancer treatment in the subject. The responses to cancer treatment in the subject can be predicted by comparing, to a reference value, the amount of CCR4⁻CCR6⁺ cell subpopulation in CD4⁺ T cell population of the subject.

## Description

### [Technical Field]

The present invention relates to the field of predicting an effect of cancer therapy. More specifically, the present invention relates to a method of predicting responsiveness of a subject to cancer therapy based on T cell composition of the subject.

### [Background Art]

A suitable therapeutic method is selected in accordance with the characteristics of a cancer patient in cancer therapy. In particular, cancer immunotherapy has drawn interest in recent years as having fewer side effects and a greater effect compared to conventional anticancer therapy targeting metabolism of cancer cells, etc. (alkylating agents, platinum formulations, antimetabolites, topoisomerase inhibitors, microtubule polymerization inhibitors, microtubule depolymerization inhibitors, etc.). Among cancer immunotherapy, anti-PD-1 immune checkpoint inhibition is a therapy that has drawn particularly significant interest.

An anti-PD-1 antibody nivolumab is superior to docetaxel, which was conventionally the standard therapy as a secondary therapy for non-small cell lung cancer by a large margin in all survival periods to be the standard therapy with a recommendation level of A in the Lung Cancer Society Guidelines (Non Patent Literature 1). Pembrolizumab, which is also an anti-PD-1 antibody, is superior to cytotoxic anticancer agents, which were conventionally the standard therapy in primary therapy, in all survival periods (in patients with expression of PD-L1 on tumor cells at 50% or greater). It has been decided that this will be the standard therapy for non-small cell lung cancer in the future.

Currently, the effect of anti-PD-1 antibodies is not limited to lung cancer. The effect is about to be proven in many types of cancer. Renal cancer, urothelial cancer, head and neck cancer, gastrointestinal cancer, malignant lymphoma, breast cancer, small cell lung cancer, colon cancer, and malignant pleural mesothelioma are covered under insurance in Japan, and hepatocellular carcinoma, ovarian cancer, and cervical cancer are expected to be covered in the future.

While anti-PD-1 antibodies appear to have achieved significant clinical success, anti-PD-1 antibodies in fact have significant problems. "Non-responder group", whose condition worsens within three months in almost all anti-PD-1 antibody clinical trials, is found from data for progression free survival (PFS). Meanwhile, in groups for which anti-PD-1 antibodies were effective for 1 year or longer, exacerbation in conditions was hardly observed thereafter, thus revealing that a state close to being healed is attained. This suggests the presence of three different subgroups, i.e., "non-responder group", "responder group" and "intermediate group" in terms of clinical effects, but a biomarker for the prediction thereof is not known. Administration of anti-PD-1 antibodies, which are expected to be the standard therapy in almost all cancer and tumor, to non-responder groups accounting for about 40% would be not only a medical problem, but also a problem for medical economics.

The inventors have already discovered that the three groups, with a therapeutic effect from cancer immunotherapy (e.g., anti-PD-1 therapy or anti-PD-L1 therapy) of progressive disease (PD), stable disease (SD), and response (complete response (CR) and partial response (PR)), each exhibit different immunological conditions, and have provided a method of predicting a response to cancer immunotherapy as either progressive disease (PD), stable disease (SD), or response (complete response (CR) and partial response (PR)) when cancer immunotherapy is administered to a subject (Patent Literature 1).

### [Citation List]

### [Patent Literature]

[PTL 1] Japanese Patent No. 6664684

### [Non Patent Literature]

[NPL 1] Brahmer J, et al. N Engl J Med 2015; 373: 123-135

### [Summary of Invention]

### [Solution to Problem]

In view of such a circumstance, the present invention has discovered a marker for predicting the effect of cancer therapy on a patient by evaluating the immune status of a patient. Since the composition of cell subpopulations in a sample obtained from a subject is used as an indicator for predicting a response of the subject to cancer therapy, it was discovered that composition of cell subpopulations that is different from conventionally known composition can be a new indicator for predicting a response to cancer therapy.

Specifically, the inventors discovered that predictive performance exceeding those of conventional indicators for predicting a response to cancer therapy can be achieved by measuring the ratio of CCR6⁺CCR4⁻ cells (preferably the ratio of CD62L^{low}CCR6⁺CCR4⁻ cells) to the entire CD4 T cells, and the ratio of CCR6⁺CCR4⁻ cells can be used to achieve the same predictive performance as the ratio of CD62L^{low}CCR6⁺CCR4⁻ cells. The present invention has been conceived based in part from the inventors discovering that responsiveness to cancer therapy is associated with the T cell composition in a subject and the composition can be utilized as a biomarker. Thus, the biomarkers of the invention are highly sensitive, specific, and convenient.

The inventors have discovered that progression free survival (PFS) after treatment with cancer immunotherapy (e.g., anti-PD-1 therapy or anti-PD-L1 therapy) and specific T cell composition exhibit significant correlation. In one embodiment of the invention, this provides a method for predicting a response to cancer immunotherapy when such cancer immunotherapy is administered to a subject. For example, the inventors also discovered that progression free survival (PFS) after treatment with cancer therapy other than cancer immunotherapy such as radiation therapy or molecularly targeted drug therapy and specific T cell composition exhibit significant correlation. In one embodiment of the invention, this provides a method for predicting a response to cancer therapy when such cancer therapy is administered to a subject.

Therefore, the present invention provides the following.
(Item 1) A method of using a relative amount of a CCR4⁻CCR6⁺ cell subpopulation in a CD4⁺ T cell population of a subject as an indicator for predicting a response of the subject to cancer therapy, comprising
   determining the relative amount of the cell subpopulation in a sample derived from the subject,
   wherein the relative amount is used as an indicator for predicting a response of the subject to cancer therapy.
(Item 2) The method of item 1, wherein comparison of the relative amount with a reference value is used as an indicator for predicting a response of the subject to cancer therapy.
(Item 3) The method of item 1 or 2, wherein comparison of the relative amount with a reference value is used as an indicator for predicting long-term survival of the subject.
(Item 4) The method of any one of items 1 to 3, wherein comparison of the relative amount with a non-responder group threshold value is used as an indicator for predicting whether the subject is a part of a non-responder group to the cancer therapy.
(Item 5) The method of any one of items 1 to 4, wherein the CCR4⁻CCR6⁺ cell subpopulation is also a CXCR3⁺ cell subpopulation.
(Item 6) The method of any one of items 1 to 4, wherein the CCR4⁻CCR6⁺ cell subpopulation is also a CXCR3⁻ cell subpopulation.
(Item 7) The method of any one of items 1 to 6, wherein the CCR4⁻CCR6⁺ cell subpopulation is also a FoxP3⁻ cell subpopulation.
(Item 8) The method of any one of items 1 to 7, wherein the CCR4⁻CCR6⁺ cell subpopulation is also a CD62L^{low} cell subpopulation.
(Item 9) The method of any one of items 1 to 7, wherein the CCR4⁻CCR6⁺ cell subpopulation is also a CD45RA⁻ cell subpopulation.
(Item 10) The method of any one of items 1 to 9, wherein the cancer therapy comprises cancer immunotherapy, radiation therapy, molecularly targeted drug therapy, surgical operation, cell infusion, or any combination thereof.
(Item 11) The method of any one of items 1 to 10, wherein the cancer therapy is cancer immunotherapy.
(Item 12) The method of item 11, wherein the cancer immunotherapy comprises administration of an immune checkpoint inhibitor.
(Item 13) The method of item 12, wherein the immune checkpoint inhibitor comprises an inhibitor selected from the group consisting of a PD-1 inhibitor, a PD-L1 inhibitor, and a CTLA-4 inhibitor.
(Item 14) The method of item 12, wherein the immune checkpoint inhibitor comprises a combination of a PD-1 inhibitor or PD-L1 inhibitor and a CTLA-4 inhibitor.
(Item 15) The method of item 13 or 14, wherein the PD-1 inhibitor is an anti-PD-1 antibody that inhibits an interaction between PD-1 and PD-L1.
(Item 16) The method of any one of items 13 to 15, wherein the PD-1 inhibitor comprises nivolumab or pembrolizumab.
(Item 17) The method of any one of items 13 to 16, wherein the PD-L1 inhibitor is an anti-PD-L1 antibody that inhibits an interaction between PD-1 and PD-L1.
(Item 18) The method of item 17, wherein the PD-L1 inhibitor comprises durvalumab, atezolizumab, and avelumab.
(Item 19) The method of any one of items 13 to 18, wherein the CTLA-4 inhibitor comprises ipilimumab and tremelimumab.
(Item 20) The method of any one of items 4 to 19, wherein the non-responder group threshold value is determined by considering sensitivity and specificity for detection of a non-responder group.
(Item 21) The method of any one of items 4 to 20, wherein the non-responder group threshold value is determined so that sensitivity for detection of a non-responder group exceeds about 90%.
(Item 22) The method of any one of items 4 to 21, wherein the non-responder group threshold value is determined so that specificity for detection of a non-responder group exceeds about 90%.
(Item 23) The method of any one of items 1 to 22, wherein a relative amount of the cells is measured using a peripheral blood sample.
(Item 24) The method of any one of items 2 to 23, wherein the relative amount being greater than or equal to the reference value indicates that the subject is responsive to the cancer therapy.
(Item 25) A composition for treating cancer in a subject, comprising an immune checkpoint inhibitor, wherein the subject is a subject predicted to be responsive to cancer immunotherapy by the method of any one of items 11 to 24.
(Item 26) The composition of item 25, wherein the immune checkpoint inhibitor comprises an inhibitor selected from the group consisting of a PD-1 inhibitor, a PD-L1 inhibitor, and a CTLA-4 inhibitor.
(Item 27) The composition of item 26, wherein the immune checkpoint inhibitor comprises a combination of a PD-1 inhibitor or PD-L1 inhibitor and a CTLA-4 inhibitor.
(Item 28) The composition of item 26 or 27, wherein the PD-1 inhibitor is an anti-PD-1 antibody that inhibits an interaction between PD-1 and PD-L1.
(Item 29) The composition of any one of items 26 to 28, wherein the PD-1 inhibitor comprises nivolumab or pembrolizumab.
(Item 30) The composition of any one of items 26 to 27, wherein the PD-L1 inhibitor is an anti-PD-L1 antibody that inhibits an interaction between PD-1 and PD-L1.
(Item 31) The composition of item 30, wherein the PD-L1 inhibitor comprises durvalumab, atezolizumab, or avelumab.
(Item 32) The composition of any one of claims 26 to 31, wherein the CTLA-4 inhibitor comprises ipilimumab.
(Item 33) A kit for predicting a response of a subject to cancer therapy, comprising a CD4 detecting agent, a CCR4 detecting agent, and a CCR6 detecting agent, wherein the prediction is performed by using a relative amount of a CCR4⁻CCR6⁺ cell subpopulation in a CD4⁺ T cell population of the subject as an indicator for predicting a response of the subject to cancer therapy.
(Item 34) The kit of item 33, wherein comparison of the relative amount with a reference value is used as an indicator for predicting a response of the subject to cancer therapy.
(Item 35) The kit of item 33 or 34, wherein comparison of the relative amount with a reference value is used as an indicator for predicting long-term survival of the subject.
(Item 36) The kit of any one of items 33 to 35, wherein comparison of the relative amount with a non-responder group threshold value is used as an indicator for predicting whether the subject is a part of a non-responder group to the cancer therapy.
(Item 37) The kit of any one of items 33 to 36, wherein the detecting agent is an antibody.
(Item 38) The kit of any one of items 33 to 37, further comprising a CXCR3 detecting agent.
(Item 39) The kit of any one of items 33 to 38, wherein the cancer therapy comprises cancer immunotherapy, radiation therapy, molecularly targeted drug therapy, surgical operation, cell infusion, or any combination thereof.
(Item 40) The kit of any one of items 33 to 39, wherein the cancer therapy is cancer immunotherapy.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

The feature and significant action/effect of the present disclosure other than those described above will be clear to those skilled in the art by referring to the following Detailed Description of the Invention section and the drawings.

### [Brief Description of Drawings]

[Figure 1] Figure **1** is a schematic diagram representing Th differentiation of human CD4 T cells.
[Figure 2] Figure **2** is a result of mapping analysis by mass cytometry of CyTOF on peripheral blood CD4⁺ T cells.
[Figure 3] Figure **3** is a result of mapping analysis by mass cytometry of CyTOF on various cell subpopulations in one embodiment of the invention.
[Figure 4] Figure **4** is a graph showing correlation between two clusters, i.e., CD62L^{low}CXCR3⁺CCR6⁺CCR4⁻ (Th1/17) and CD62L^{low}CXCR3⁻CCR6⁺CCR4⁻ (CCR6 SP), and progression free survival in one embodiment of the invention. The right side in Figure **4** is a combination of two clusters CD62L^{low}CXCR3⁺CCR6⁺CCR4⁻ (Th1/17) and CD62L^{low}CXCR3⁻CCR6⁺CCR4⁻ (CCR6 SP). The left side in Figure **4** is a graph showing the correlation between %CD62L^{low}/CD4⁺ and progression free survival.
[Figure 5] Figure **5** is a graph showing the correlation between %CCR6⁺CCR4⁻/CD4⁺ and progression free survival in one embodiment of the invention.
[Figure 6] Figure **6** is a graph showing the correlation between %CCR6⁺CCR4⁻/CD4⁺ and progression free survival obtained using flow cytometry analysis (Fortessa) and correlation between %CCR6⁺CCR4⁻/CD4⁺ and progression free survival obtained using CyTOF in one embodiment of the invention.
[Figure 7] Figure **7** is a graph showing the correlation between progression free survival after pembrolizumab monotherapy and results of Th analysis by CyTOF in one embodiment of the invention.
[Figure 8] Figure **8** is a graph showing the correlation between the total number of CD62L^{low} Th1/17 and CD62L^{low} CCR6 SP clusters and progression free survival.
[Figure 9] Figure **9** is a correlation graph showing that the sum of Th1/17 and CCR6 SP can be measured as a CCR4⁻CCR6⁺ cell cluster.
[Figure 10] Figure **10** is a table showing the correlation between a Th cluster and overall survival in one embodiment of the invention.
[Figure 11] Figure **11** is a graph of correlation between progression free survival and overall survival of a CCR6⁺CCR4⁻CD4 T cell cluster in comparison with other markers in one embodiment of the invention.
[Figure 12] Figure **12** is a graph showing the performance of the ratio of CCR6⁺CCR4⁻ cells in CD4⁺ T cells as an indicator for predicting overall survival in one embodiment of the invention. The right panel plots sensitivity against specificity when the threshold value is varied. The area of the region under the plotted points is 0.9628, so that this is understood as a very good marker.
[Figure 13] Figure **13** is a graph showing the correlation between PFS after chemoradiotherapy and CCR6⁺CCR4⁻ CD4 T cell cluster in one embodiment of the invention.
[Figure 14] Figure **14** is a graph showing the correlation between PFS after EGFR-TKI therapy and CCR6⁺CCR4⁻ CD4 T cell cluster in one embodiment of the invention.
[Figure 15] Figure **15** is a schematic diagram showing TCR clonotype count for each of CD62L^{low} Th1, CD62L^{low} Th1/17, CD62L^{low} CCR6 SP, CD62L^{low} Th17, and CD62L^{low} TP.
[Figure 16] Figure **16** is a graph showing that a CD62L^{low} Th1/17 + CD62L^{low} CCR6 SP cell cluster correlates with a Th1/17 + CCR6 SP cell cluster.
[Figure 17] Figure **17** is a schematic diagram representing a peripheral blood CD4⁺ T cell cluster and a chemokine receptor expressed in each fraction.
[Figure 18] Figure **18** is a table of results of multivariate analysis showing the correlation between a peripheral blood CD4⁺ T cell cluster and PFS after anti-PD-1 antibody therapy.
[Figure 19] Figure **19** is a diagram showing results of DNA methylome (methylation) analysis and pseudotemporal analysis of gene expression of a peripheral blood CD4⁺ T cell cluster.
[Figure 20] Figure **20** is a diagram showing the correlation of PFS with Th1/17 and CCR6 SP in each therapy line.
[Figure 21] Figure **21** is a diagram showing the correlation between CD4⁺ T cells in a tumor microenvironment and PFS.
[Figure 22] Figure **22** is a result of network analysis on a cell cluster in peripheral blood and a cell cluster in a tumor microenvironment (TME).
[Figure 23] Figure **23** is a graph showing the change in a cell cluster in peripheral blood before and after surgery.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or the detailed basic technology that are particularly used herein are described hereinafter as appropriate.

As used herein, "about" refers to a range of ± 10% from the numerical value that is described subsequent to "about".

As used herein, "biomarker" refers to a characteristic that can be objectively measured and evaluated as an indicator of a normal biological process, pathological process, or a pharmacological response to therapeutic intervention.

As used herein, "cancer" refers to malignant tumor, which is highly atypic, expands faster than normal cells, and can destructively infiltrate or metastasize surrounding tissue, or the presence thereof. In the present invention, cancer includes solid cancer and hematopoietic tumor. Examples thereof include, but are not limited to, non-small cell lung cancer, small cell lung cancer, renal cancer, Hodgkin's disease, head and neck cancer, breast cancer, gastric cancer, malignant melanoma, colon cancer, acute myeloid leukemia, chronic myeloid leukemia, acute lymphatic leukemia, gastrointestinal stromal tumor, pancreatic neuroendocrine tumor, skin cancer, etc.

As used herein, "cancer immunotherapy" refers to a method of treating cancer using a biological defense mechanism such as the immune mechanism of an organism.

As used herein, "antitumor immune response" refers to any immune response against tumor in an organism.

As used herein, "correlation" refers to two matters having a statistically significant correlation. For example, "relative amount of B correlated with A" refers to the relative amount of B being statistically significantly affected (e.g., increase or decrease) when A occurs.

As used herein, "immune activation" refers to enhancement in the immune function for eliminating foreign objects in the body. Immune activation can be indicated by an increase in the amount of any factor (e.g., immune cell or cytokine) that has a positive effect on immune function.

As used herein, "cell subpopulation" refers to any group of cells with some type of a common feature in a cell population including cells with diverse properties. For cell subpopulations with a specific name that is known in the art, a specific cell subpopulation can be mentioned by using such a term or by describing any property (e.g., expression of a cell surface marker).

As used herein, the term "relative amount" with regard to cells can be interchangeably used with "ratio". Typically, the terms "relative amount" and "ratio" refer to the number of cells constituting a given cell subpopulation (e.g., CCR4⁻CCR6⁺ cell subpopulation) with respect to the number of cells constituting a specific cell population (e.g., CD4⁺T cell population).

As used herein, "sensitivity" refers to the ratio of the number of subjects with a given feature among selected subjects to the total number of subjects with a given feature in a subject population when selecting a subject with a given feature in a population of subjects. If, for example, subjects with a given feature in a population of subjects are all selected, sensitivity is 100%. If half of the subjects with a given feature in a population of subjects is selected, sensitivity is 50%. If a subject with a given feature in a population of subjects is not selected at all, sensitivity is 0%. Sensitivity is determined as, for example, (number of subjects with a given feature in selected subjects)/(total number of subjects with a given feature in a subject population) . When it is desirable to find subjects in a certain state (e.g., long-term survival as a result of cancer immunotherapy), determination with high sensitivity means that such subjects are likely determined to be in such a state with certainty.

As used herein, "specificity" refers to the ratio of the number of subjects with a given feature among selected subjects to the total number of selected subjects when selecting a subject with a given feature in a subject population. If, for example, candidates selected from a population of subjects all have a given feature, specificity is 100%. If half of the candidates selected from a population of subjects has a given feature, specificity is 50%. If none of the candidates selected from a population of subjects has a given feature, specificity is 0%. Specificity is determined as, for example, (number of subjects with a given feature in selected subjects)/(total number of selected subjects). Determination with high specificity means that the probability of incorrectly determining a subject who is not in a certain state (e.g., responder to cancer immunotherapy) to be in another state (e.g., long-term survival as a result of caner immunotherapy) is low.

As used herein, "progression free survival (PFS)" refers to a period in which cancer is stable without progression during or after therapy, i.e., the state of disease does not progress. As used herein, "progression free survival (PFS)" includes any period in which cancer is clinically determined to be in a stable state without progression or the state of disease is determined to be without progression.

As used herein, "non-responder group" refers to a group of subjects determined as progressive disease (PD) in the early stage by about 9 weeks after starting therapy when the therapeutic effect from undergoing cancer therapy is determined in accordance with RECIST ver 1.1. A non-responder group is also referred to as a PD group, progressive group, or NR (Non-responder), which are interchangeably used herein.

As used herein, "partial responder group" refers to a group of subjects determined as partial response (PR) when the therapeutic effect from undergoing cancer therapy is determined in accordance with RECIST ver 1.1. A partial responder group is also referred to as a PR group, which is interchangeably used herein.

As used herein, "relative value" refers to a value obtained by calculating a certain value while using another value as a baseline of comparison.

As used herein, the term "detecting agent" broadly refers to any agent that is capable of detecting a substance of interest (e.g., cell surface marker or the like).

As used herein, the "amount" of a certain cell subpopulation encompasses the absolute number of certain cells and relative amount of a ratio in a cell population.

As used herein, "reference" or "reference value" refers to the amount that is the baseline of comparison for determining the increase/decrease in the amount of a marker described herein. When determining the increase/decrease of a certain amount after a certain treatment (e.g., cancer immunotherapy) relative to before the certain treatment, "reference value" can be, for example, said amount before treatment. A reference or reference value can be in a form of a given amount or relative amount of cells that is known to indicate a certain quantity (e.g., progression free survival).

As used herein, "threshold value" refers to a value that is set for a variable, which gives some type of a meaning when the variable is greater than or less than the threshold value. A threshold value is also referred to as a cut-off value herein.

As used herein, "non-responder group threshold value" refers to a threshold value that is used to distinguish a non-responder group from a stable group + responder group in a given population of subjects. When selecting a non-responder group in a given population of subjects, a non-responder group threshold value is selected to achieve a predetermined sensitivity and specificity.

As used herein, "responder group threshold value" refers to a threshold value that is used to distinguish a stable group and a responder group in a given population of subjects or in a given population of subjects from which a non-responder group is excluded using a non-responder group threshold value. When selecting a responder group in a given population of subjects or in a given population of subjects from which a non-responder group is excluded using a non-responder group threshold value, a responder group threshold value is selected to achieve a predetermined sensitivity and specificity.

As used herein, "long-term survival" indicates that progression free survival is of a certain period or longer. Long-term survivor refers to, for example, a patient without exacerbation over a certain period or longer after nivolumab therapy. Since a patient estimated to be long-term survivor is predicted to be responsive over a long period of time to cancer immunotherapy, a clinical physician can determine that cancer immunotherapy should be discontinued at the minimum length (e.g., after one administration). As used herein, a period that can be determined as long-term survival is not particularly limited and may vary depending on the attribute of the patient (age, sex, medical history, etc.), type of cancer, status of dosing, medical record, etc. For example, the period may be 500 days or longer, 400 days or longer, 300 days or longer, 150 days or longer, etc. A period that can be determined as long term survival for elderly patients may be shorter than a period that can be determined as long-term survival for young patients. Moreover, a period that can be determined as long-term survival in patients with complication with other diseases may be shorter than a period that can be determined as long-term survival in patients without complications.

As used herein, "flow cytometry" refers to a technology of measuring the number of cells, individuals, and other biological particles suspended in a liquid and individual physical/chemical/biological attributes. Results of flow cytometry can be typically expressed as a dot plot, with FSC in the X axis and SSC in the Y axis. Each cell is indicated by a dot (point) in a diagram. The position thereof is determined by the relative values of FSC and SSC. Lymphocytes which have a relatively small size and simple internal structure are displayed on the bottom left section, granulocytes which have a large size and granules inside are displayed on the top right section, and monocytes which have a large size but a simple internal structure are displayed between lymphocytes and granulocytes, with each forming a population separated from one another. The results of flow cytometry can be represented by a histogram, dot plot, etc.

As used herein, "effector cells" refer to naive cells, which have not been subjected to antigen stimulation, differentiating into immune cells that have actually acquired a function associated with immunity. In particular, three types thereof, i.e., Th1, Th2, and Th17, are mainly known for human helper T cells. Each can release a characteristic cytokine such as interferon γ (IFNγ) or interleukin 17 (IL-17). While each Th subset has a characteristic transcription factor, e.g., Th1 expresses T-bet, Th2 expresses GATA-3, etc., there are cells that express both T-bet and GATA-3. Such cells are expressed as "Th1/Th2" or "Th1/2". As used herein, "Th1/Th17" or "Th1/17" expresses both T-bet which is characteristic to Th1 and RORγt which is characteristic to Th17.

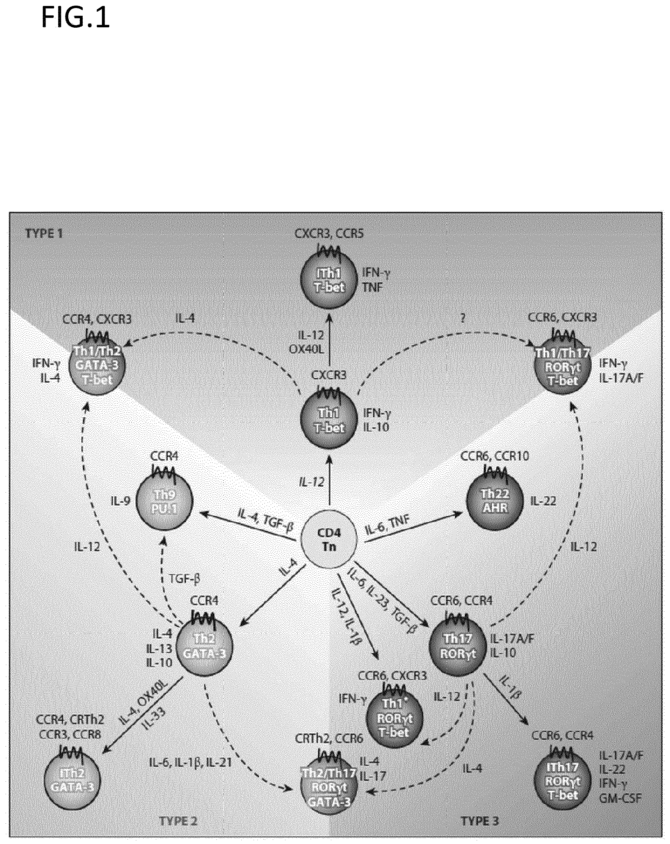

Effector cells such as Th1, Th2, and Th17 can also be classified by the type of chemokine receptor (CCR) that is expressed. For example, Th1 expresses CXCR3, Th2 expresses CCR4, and Th17 expresses CCR4 and CCR6. Classification of Th subsets can be expressed as shown in, for example, Figure **1** (Annual Review of Immunology, Vol. 34:317-334 Heterogeneity of Human CD4+ T Cells Against Microbes, Fig. 1). As used herein, "Th1/Th17" or "Th1/17" expresses CXCR3 which is characteristic to Th1, and CCR6 between the two CCRs characteristic to Th17. As used herein, CCR6 SP (single positive) is a type that was not classified in the past in Th type classification by CCR, and is a Th subset expressing only CCR6 among CCR4, CCR6, and CXCR3. As used herein, TP (triple positive) is a type that was not classified in the past in Th type classification by CCR, and is a Th subset expressing all three of CCR4, CCR6, and CXCR3.

As used herein, "cancer therapy" refers to any therapy administered to a cancer patient for removing or reducing tumor, including, but not limited to, cancer immunotherapy, radiation therapy, molecularly targeted drug therapy, surgical operation, cell infusion, and any combination thereof.

### (Cancer immunotherapy)

Cancer immunotherapy is a method of treating cancer using a biological defense mechanism of an organism. Cancer immunotherapy is largely divided into cancer immunotherapy from strengthening the immune function against cancer and cancer immunotherapy from inhibiting the immune evasion mechanism of cancer. Cancer immunotherapy further includes active immunotherapy for activating the immune function in the body and passive immunotherapy for returning immune cells with an immune function activated or the numbers thereof expanded outside the body back into the body. It is understood that the biomarker of the invention evaluates the balance of the overall CD4⁺ T-cell immunity to evaluate the tumor immunity itself as a whole, so that a therapeutic effect of all cancer immunotherapy can be broadly predicted.

Examples of cancer immunotherapy include non-specific immunopotentiators, cytokine therapy, cancer vaccine therapy, dendritic cell therapy, adoptive immunotherapy, non-specific lymphocyte therapy, cancer antigen specific T cell therapy, antibody therapy, immune checkpoint inhibition therapy, etc. The Examples in the present specification demonstrate that the biomarker of the invention accurately predicts a therapeutic effect of especially, although not limited to, immune checkpoint inhibition therapy.

PD-1 inhibitors are representative examples of immune checkpoint inhibitors. Examples of PD-1 inhibitors include, but are not limited to, anti-PD-1 antibody nivolumab (sold as Opdivo^{™}) and pembrolizumab. In one preferred embodiment, nivolumab can be selected as such an inhibitor. Although not wishing to be bound by any therapy, one of the reasons that therapy using nivolumab is preferred is because the Examples demonstrate that the use of the biomarker of the invention can clearly identify a responsive subject and a non-responsive subject, and especially because it is revealed that responsiveness and non-responsiveness can be clearly distinguished by a specific threshold value. Of course, it is understood that the biomarker of the invention can be utilized for other PD-1 inhibitors to the same degree.

The present invention can also use PD-L1 inhibitors and CTLA-4 inhibitors to the same extent as PD-1 inhibitors.

It is understood that anti-PD-1 antibodies achieve an anti-cancer effect by releasing the suppression of T-cell activation by a PD-1 signal. It is understood that anti-PD-L1 antibodies also achieve an anticancer effect by releasing the suppression of T-cell activation by a PD-1 signal. While the mechanism of PD-1 inhibiting a T-cell function is not fully elucidated, it is understood that an interaction between PD-1 (programmed death 1) and PD-L1 or PD-L2 recruits a tyrosine phosphatase, SHP-1 or 2, to the cytoplasmic domain of PD-1 and inactivate a T-cell receptor signaling protein ZAP70 to suppress activation of T-cells (Okazaki, T., Chikuma, S., Iwai, Y. et al.: A rheostat for immune responses: the unique properties of PD-1 and their advantages for clinical application. Nat. Immunol., 14, 1212-1218 (2013)). This is understood to be caused by the recruitment of SHP-1 or 2 to a part known as the ITSM motif which dephosphorylates proximal signaling kinase of a T-cell receptor in the vicinity. In other words, the memory of "being stimulated by an antigen" is erased from a T-cell that has been stimulated by an antigen.

PD-1 is expressed at a high level in killer T-cells and natural killer cells, which have infiltrated a cancer tissue. It is understood that an immune response mediated by a PD-1 signal from PD-1 is attenuated by PD-L1 on tumor. While the immune response mediated by a PD-1 signal is attenuated by PD-L1, an effect of enhancing an anti-tumor immune response is attained by inhibiting an interaction between PD-1 and PD-L1 and/or signaling induced by an interaction with an anti-PD-1 antibody.

Other examples of an immune checkpoint inhibitor include PD-L1 inhibitors (e.g., anti-PD-L1 antibodies avelumab, durvalumab, and atezolizumab), etc.

PD-L1 inhibitors bind to and inhibit the aforementioned PD-1 pathway on the PD-L1 side to inhibit an interaction between PD-1 and PD-L1 and/or signaling induced by an interaction to induce an anti-tumor immune response. Although not wishing to be bound by any therapy, subjects who are responsive or non-responsive to therapy that inhibits the PD-1 pathway (e.g., anti-PD-1 antibody or anti-PD-L1 antibody) can be clearly identified by using the biomarker of the invention.

Other examples of an immune checkpoint inhibitor include CTLA-4 inhibitors (e.g., anti-CTLA-4 antibody ipilimumab or tremelimumab).

CTLA-4 inhibitors activate T-cells to induce an anti-tumor immune response. T-cells are activated by an interaction of CD28 on the surface with CD80 or CD86. However, it is understood that surface expressed CTLA-4 (cytotoxic T-lymphocyte-associated antigen 4) preferentially interacts with CD80 or CD86 with higher affinity than CD28 to suppress activation, even for T-cells that have been activated. CTLA-4 inhibitors induce an anti-tumor immune response by inhibiting CTLA-4 to prevent inhibition of an interaction between CD28 and CD80 or CD86.

In another embodiment, an immune checkpoint inhibitor may target an immune checkpoint protein such as TIM-3 (T-cell immunoglobulin and mucin containing protein-3), LAG-3 (lymphocyte activation gene-3), B7-H3, B7-H4, B7-H5 (VISTA), or TIGIT (T cell immunoreceptor with Ig and ITIM domain).

It is understood that the aforementioned immune checkpoints suppress an immune response to autologous tissue, but immune checkpoints increase in T-cells when an antigen such as a virus is present in vivo for an extended period of time. It is understood that tumor tissue is also an antigen which is present in vivo for an extended period of time, so that an anti-tumor immune response is evaded by such immune checkpoints. The aforementioned immune checkpoint inhibitors render such an evasion function ineffective to achieve an anti-tumor effect. Although not wishing to be bound by any therapy, it is understood that the biomarker of the invention evaluates the balance of the overall anti-tumor immune responses of humans so that it can be used as an indicator for accurately predicting a therapeutic effect of such an immune checkpoint inhibitor.

One embodiment of the invention provides a composition comprising an immune checkpoint inhibitor. A composition comprising an immune checkpoint inhibitor can attain a significant therapeutic effect at a high probability by administration thereof to a subject who has been selected by evaluation with the biomarker of the invention.

The composition comprising an immune checkpoint inhibitor of the invention is generally administered systemically or locally in an oral or parenteral form.

The dosage varies depending on the age, body weight, symptom, therapeutic effect, administration method, treatment time, etc., but is generally administered, for example, orally one to several times a day in the range of 0.1 mg to 100 mg per dose per adult, or is administered parenterally (preferably intravenously) one to several times a day in the range of 0.01 mg to 30 mg per dose per adult, or continuously administered intravenously in the range of 1 hour to 24 hours per day. Of course, the dosage varies depending on various conditions, so that an amount less than the above dosage may be sufficient or an amount beyond the range may be required.

For administration, a composition comprising an immune checkpoint inhibitor can have a dosage form such as a solid agent or liquid agent for oral administration or an injection, topical agent, or suppository for parenteral administration. Examples of solid agents for oral administration include tablets, pills, capsules, powder, granules, etc. Capsules include hard and soft capsules.

The composition of the invention includes one or more active ingredients (e.g., antibody to an immune checkpoint protein), which is directly used or is mixed or formulated with an excipient (lactose, mannitol, glucose, microcrystalline cellulose, starch, etc.), binding agent (hydroxypropyl cellulose, polyvinyl pyrrolidone, magnesium aluminometasilicate, etc.), disintegrant (calcium cellulose glycolate, etc.), lubricant (magnesium stearate, etc.), stabilizer, solubilizing agent (glutamic acid, aspartic acid, etc.) in accordance with a conventional method for use as needed. The composition may also be coated with a coating agent (refined sugar, gelatin, hydroxypropyl cellulose, hydroxypropyl methyl cellulose phthalate, etc.) or coated with two or more layers as needed. Capsules made of a substance that can be absorbed such as gelatin are also encompassed.

The composition of the invention comprises a pharmaceutically acceptable aqueous agent, suspension, emulsion, syrup, elixir, etc. when formulated as a liquid agent for oral administration. In such a liquid agent, one or more active ingredients is dissolved, suspended, or emulsified in a commonly used diluent (purified water, ethanol, a mixture thereof, etc.). Such a liquid agent may also contain a humectant, suspending agent, emulsifier, sweetener, flavor, fragrance, preservative, buffer, etc.

Examples of injections for parenteral administration include a solution, suspension, emulsion, and solid injection that is used by dissolving or suspending it in a solvent at the time of use. An injection is used by dissolving, suspending, or emulsifying one or more active ingredients into a solvent. Examples of solvents that are used include distilled water for injections, saline, vegetable oil, propylene glycol, polyethylene glycol, alcohols such as ethanol, combination thereof, etc. Such an injection may also comprise a stabilizer, solubilizing agent (glutamic acid, aspartic acid, polysorbate 80^{™}, etc.), suspending agent, emulsifier, analgesic, buffer, preservative, etc. They are sterilized in the final step or prepared through an aseptic operation. It is also possible to manufacture an aseptic solid agent such as a lyophilized product, which can be sterilized before use or dissolved in aseptic distilled water for injection or another solvent for use.

### (Cancer)

Examples of target cancer in the present invention include, but are not limited to, melanoma (malignant melanoma), non-small cell lung cancer, renal cell cancer, malignant lymphoma (Hodgkin's or non-Hodgkin's lymphoma), head and neck cancer, urological cancer (bladder cancer, urothelial cancer, and prostate cancer), small cell lung cancer, thymic carcinoma, gastric cancer, esophageal cancer, esophagogastric junction cancer, liver cancer (hepatocellular carcinoma and intrahepatic cholangiocarcinoma), primary brain tumor (glioblastoma and primary central nervous system lymphoma), malignant pleural mesothelioma, gynecologic cancer (ovarian cancer, cervical cancer, and uterine cancer), soft tissue sarcoma, cholangiocarcinoma, multiple myeloma, breast cancer, colon cancer, etc.

### (Fractionation/separation of cells)

A sample for fractionation/separation of T cells can be suitably collected from a subject using a conventional method. For example, such a sample can be collected from peripheral blood, bone marrow, tumor tissue, hematopoietic tissue, spleen, normal tissue, lymph, etc. of a subject. Sample collection from peripheral blood can be advantageous for being simple and non-invasive.

The composition of T cells in a sample of a subject can be measured by those skilled in the art using a conventional method. Generally, the number of cells that are positive for a marker (e.g., CD4) defining a cell subpopulation of interest in a sample can be measured using flow cytometry or the like. The measurement of the composition of a cell population generally uses flow cytometry, but other means may also be used, such as immunostaining on a sample comprising cells, a method using an antibody array, protein expression analysis in a sample comprising cells (e.g., Western blot, mass spectrometry, HPLC, etc.), or mRNA expression analysis in a sample comprising cells (microarray, next generation sequencing, etc.).

To measure the cell count in each cell subpopulation such as a CXCR3⁺CCR6⁺CCR4⁻ T-cell subpopulation and CXCR3⁻ CCR6⁺CCR4⁻ T-cell subpopulation, the cell count may be found by experimentally excluding cells other than subpopulations of each kind from all cells. For example, cells corresponding to a T-cell subpopulation of interest can be separated from peripheral blood without using a given antibody by using a CD4⁺ Effector Memory T cell isolation kit, human (Militenyi Biotech), etc. For example, cells corresponding to a CD4⁺CD62L^{low} T cell subpopulation can be separated from peripheral blood without using a CD4 antibody and CD62L antibody when a CD4⁺ Effector Memory T cell isolation kit, human (Militenyi Biotech) is used. The total viable cell count can be counted and recorded, and the number of cells obtained using this kit can be counted and recorded. When a CD4⁺CD25⁺ Regulatory T cell isolation kit, human (Militenyi Biotech) is used, the number of cells corresponding to a CD4⁺CD25⁺CD4⁺Foxp3⁺CD25⁺ T cell subpopulation can be found without using an anti-FoxP3 antibody. Since FoxP3 is localized in the nucleus in cells, this has an advantage of eliminating a step for staining a molecule in the nucleus. As a similar kit, CD4⁺CD25⁺CD127^{dimi/-}Regulatory T cell isolation kit, human (Militenyi Biotech) or CD25⁺CD49d⁻ Regulatory T cell isolation kit, human (Militenyi Biotech) can also be selected. In one embodiment of the invention, any kit that can measure the cell count of each T cell subpopulation of interest can be used.

An antibody does not need to be used. Antibodies that can specifically recognize and bind a molecule expressed on individual cells are prepared so that they can emit color when bound to a molecule expressed on the cell surface or inside the cells. The antibodies are then detected to measure the number of cells that are emitting color. Since the molecules expressed on the cell surface or inside the cells are proteins, mRNA encoding a protein when the protein is expressed is also formed in the cells. In other words, it is sufficient to examine mRNA in individual cells to examine the presence/absence of mRNA encoding a protein of interest. This is made possible by single cell gene expression analysis, i.e., mRNA analysis at a single cell level. Examples of single cell gene expression analysis include 1) a method of next generation sequencing using Quartz-Seq, 2) a method of isolating cells using a Fluidigm C1 System or ICELL8 Single-Cell System to prepare a library with SMART-Seq v4, 3) a method of separating cells with a cell sorter and measuring the cells by quantitative PCR using an Ambion Single Cell-to-CT kit, 4) CyTOF SYSTEM (Helios), etc.

Specifically, blood is obtained, viable cells are counted, and cells are separated with a cell sorter or the like. For example, Ambion Single Cell-to-CT kit can be used on the individual separated cells to measure the expression level of a specific gene with an apparatus for quantitative PCR. Based on the result, individual cells are examined as to which subpopulation such as the CXCR3⁺CCR6⁺CCR4⁻ or CXCR3⁻CCR6⁺CCR4⁻ T cell subpopulation the cells fall under to count the number of cells falling under each subpopulation. Examples of candidate genes whose expression is examined include, but are not limited to, αβTCR, CD3, CD4, CD25, CTLA4, GITR, FoxP3, STAT5, FoxO1, FoxO3, IL-10, TGFbeta, IL-35, SMAD2, SMAD3, SMAD4, CD62L, CD44, IL-7R (CD127), IL-15R, CCR7, BLIMP1, etc. Candidates can be appropriately changed in accordance with the cell subpopulation of interest.

Measurement of the ratio of cell subpopulations or comparison with a reference value or threshold value in the present invention may use a standard sample having a defined signal. Signals can be compared between a standard (e.g., particle to which a fluorescent pigment is attached) prepared to induce a fluorescent signal corresponding to a given cell subpopulation and a sample comprising a cell population to measure the amount or ratio of a cell subpopulation in the sample by comparison with a standard. Signals can also be compared between a standard (e.g., particle to which a fluorescent pigment is attached) prepared to induce a fluorescent signal corresponding to a predetermined reference value or threshold value and a sample comprising a cell population to determine the presence/absence or the amount of the marker of the invention in the T cell composition in the sample by comparison with a standard.

When determining a specific marker to be high (high expression) or low (low expression) in the present invention, those skilled in the art can use a reference classification for expression intensity that is commonly used in the art. For example, it is possible to clearly divide CD62L into CD62L^{low} and CD62L^{high} using the signal intensity corresponding to a 10E2 signal when using a PE-labeled anti-human CD62L antibody as the boundary (WO 2018/147291) .

In one embodiment of the invention, those skilled in the art can suitably identify a surface marker of the cells shown to fractionate or count the cells.

CD4 T cells are known to change into several differentiation states as effector cells, including Th1 which mainly produces IFNγ and is strongly associated with cellular immunity, Th2 which mainly produces IL-4 and IL-5 and is associated with allergies, Th17 which is known to be associated with autoimmune diseases, etc. and produces IL-17, etc. CD4 T cells differentiated into each state are known to express a characteristic chemokine receptor (CCR). The Th type can be classified by the type of CCR expressed. Th differentiation of human CD4 T cells is known to be more complex compared to mice, etc., and can be classified, for example as shown in Figure 1 (Annual Review of Immunology, Vol. 34: 317-334 Heterogeneity of Human CD4+ T Cells Against Microbes, Fig. 1).

### (Preferred embodiments)

One aspect of the invention provides a method of using a relative amount of a CCR4⁻CCR6⁺ cell subpopulation in a CD4⁺ T cell population of a subject as an indicator for predicting a response of the subject to cancer therapy. In one embodiment, the method of the invention comprises determining the relative amount of the cell subpopulation in a sample derived from the subject, wherein comparison of the relative amount with a reference value is used as an indicator for predicting a response of the subject to cancer therapy. In another embodiment, comparison of the relative amount with a reference value is used as an indicator for predicting long-term survival of the subject.

Another aspect of the invention provides a method of using an amount of a CCR4⁻CCR6⁺CD4⁺ T cell subpopulation of a subject as an indicator for predicting a response of the subject to cancer therapy. In one embodiment, the method of the invention comprises determining the amount of the cell subpopulation in a sample derived from the subject, wherein comparison of the amount with a reference value is used as an indicator for predicting a response of the subject to cancer therapy. In another embodiment, comparison of the amount with a reference value is used as an indicator for predicting long-term survival of the subject.

An aspect of the invention provides a method of using a relative amount of a CCR4⁻CCR6⁺ cell subpopulation in a CD4⁺ T cell population of a subject as an indicator for predicting a response of the subject to cancer therapy, wherein comparison of the relative amount of the cell subpopulation in a sample derived from the subject with a non-responder group threshold value is used as an indicator for predicting whether the subject is a part of a non-responder group to the cancer therapy.

Another aspect of the invention provides a method of using an amount of a CCR4⁻CCR6⁺CD4⁺ T cell subpopulation of a subject as an indicator for predicting a response of the subject to cancer therapy, wherein comparison of the amount of the cell subpopulation in a sample derived from the subject with a non-responder group threshold value is used as an indicator for predicting whether the subject is a part of a non-responder group to the cancer therapy.

In one embodiment of the invention, the amount or relative amount which is greater than or equal to a non-responder group threshold value can indicate that the subject is not part of a non-responder group to the cancer therapy. In another embodiment, the amount or relative amount which is greater than or equal to a reference value can indicate that the subject is responsive to the cancer therapy or is in long-term progression free survival after cancer therapy.

Another aspect of the invention provides a method of using a relative amount of a CCR4⁻CCR6⁺ cell subpopulation in a CD4⁺ T cell population of a subject as an indicator for predicting a response of the subject to cancer immunotherapy. In one embodiment, the method of the invention comprises determining the relative amount of the cell subpopulation in a sample derived from the subject, wherein comparison of the relative amount with a reference value is used as an indicator for predicting a response of the subject to cancer immunotherapy. In another embodiment, comparison of the relative amount with a reference value is used as an indicator for predicting long-term survival of the subject.

Another aspect of the invention provides a method of using an amount of a CCR4⁻CCR6⁺CD4⁺ T cell subpopulation of a subject as an indicator for predicting a response of the subject to cancer immunotherapy. In one embodiment, the method of the invention comprises determining the amount of the cell subpopulation in a sample derived from the subject, wherein comparison of the amount with a reference value is used as an indicator for predicting a response of the subject to cancer immunotherapy. In another embodiment, comparison of the amount with a reference value is used as an indicator for predicting long-term survival of the subject.

An aspect of the invention provides a method of using a relative amount of a CCR4⁻CCR6⁺ cell subpopulation in a CD4⁺ T cell population of a subject as an indicator for predicting a response of the subject to cancer immunotherapy, wherein comparison of the relative amount of the cell subpopulation in a sample derived from the subject with a non-responder group threshold value is used as an indicator for predicting whether the subject is a part of a non-responder group to the cancer immunotherapy.

Another aspect of the invention provides a method of using an amount of a CCR4⁻CCR6⁺CD4⁺ T cell subpopulation of a subject as an indicator for predicting a response of the subject to cancer immunotherapy, wherein comparison of the amount of the cell subpopulation in a sample derived from the subject with a non-responder group threshold value is used as an indicator for predicting whether the subject is a part of a non-responder group to the cancer immunotherapy.

In one embodiment of the invention, the amount or relative amount which is greater than or equal to a non-responder group threshold value can indicate that the subject is not part of a non-responder group to the cancer immunotherapy. In another embodiment, the amount or relative amount which is greater than or equal to a reference value can indicate that the subject is responsive to the cancer immunotherapy or is in long-term progression free survival after cancer immunotherapy.

It was discovered in the present invention that the amount of a CCR4⁻CCR6⁺CD4⁺ T cell subpopulation of a subject or a relative amount thereof is proportional to progression free survival after cancer therapy such as cancer immunotherapy, radiation therapy, or molecularly targeted drug administration, as shown in the Examples. For this reason, in one embodiment, an amount of a CCR4⁻CCR6⁺CD4⁺ T cell subpopulation of a subject or a relative amount thereof which is high can predict that the subject is responsive to cancer therapy such as cancer immunotherapy (especially cancer immunotherapy using an immune checkpoint inhibitor), radiation therapy, molecularly targeted drug administration, or surgical operation.

Thus, the present invention can compare the amount of a cell subpopulation with a suitable reference and predict long-term survival (e.g., progression free survival of 500 days or longer after cancer immunotherapy) from cancer therapy such as cancer immunotherapy, radiation therapy, molecularly targeted drug administration, and surgical operation of a subject by the comparison. For example, an increase in the amount of a cell subpopulation in a sample to more than a reference corresponding to the amount of a CCR4⁻CCR6⁺CD4⁺ T cell subpopulation indicating 500 days of progression free survival can indicate that long-term survival of the subject is predicted in cancer therapy such as cancer immunotherapy, radiation therapy, molecularly targeted drug administration, or surgical operation. Alternatively, the amount of a cell subpopulation in a sample not increased to more than a reference can indicate that long-term survival of the subject is not predicted in cancer therapy such as cancer immunotherapy, radiation therapy, molecularly targeted drug administration, or surgical operation.

Examples of a reference or reference value include, but are not limited to, the corresponding amount of a cell subpopulation in a sample of a subject before cancer therapy such as cancer immunotherapy, radiation therapy, molecularly targeted drug administration, or surgical operation. In addition, a value experimentally calculated from a sample of a subject who has not undergone cancer therapy such as cancer immunotherapy, radiation therapy, molecularly targeted drug administration, or surgical operation, etc. can also be used as a reference. A value indicating a given progression free survival can also be used as a reference or reference value.

An increase relative to a reference can be indicated by an amount of cell subpopulation after cancer immunotherapy, which is an amount exceeding the reference, an amount that is 1, 2, 3, 4, 5, 10, 15, 20, or 30% beyond the reference, or an amount that is more than 1.5-fold, 2-fold, 3-fold, or 5-fold of the reference. Typically, the amount is considered to be increased relative to the reference if the amount exceeds the reference value. When the reference is experimentally calculated, the amount can be considered to be increased relative to the reference if an increase exceeding a suitable error relative to the reference value is observed. Examples of suitable errors include 1 standard deviation, 2 standard deviations, 3 standard deviations, and greater errors.

The relative amount of a specific cell subpopulation of the invention in a CD4⁺ T cell population of a subject can be used for the detection of a long-term survival group, detection of a non-responder group, detection of a responder group, and/or detection of a stable group. Detection of these groups can be achieved by comparing a relative amount of a specific cell subpopulation of the invention in a CD4⁺ T cell population of a subject with a threshold value.

A threshold value can be determined by considering sensitivity and specificity. Sensitivity and specificity can be sensitivity and specificity for the detection of a long-term survival group, detection of a non-responder group, detection of a responder group, or detection of a stable group. In one embodiment, a threshold value at which sensitivity and specificity are both 100% can be set for the biomarker of the invention. When two or more indicators disclosed as a biomarker of the invention are used, threshold values can be determined for each indicator and used and distinguished as a first threshold value, second threshold value, third threshold value, and fourth threshold value as needed.

A threshold value can be determined so that sensitivity for the detection of a long-term survival group, detection of a non-responder group, detection of a responder group, or detection of a stable group exceeds about 90%. In another embodiment, a threshold value can be determined so that the sensitivity for the detection of a non-responder group, detection of a responder group, or detection of a stable group is about 100%. In still another embodiment, a threshold value can be determined so that specificity for the detection of a non-responder group, detection of a responder group, or detection of a stable group exceeds about 90%. In still another embodiment, a threshold value can be determined so that specificity for the detection of a non-responder group, detection of a responder group, or detection of a stable group is about 100%

It is understood that the biomarker of the invention is for evaluating the balance of the overall antitumor immune responses including CD4⁺ T cells, dendritic cells, and/or CD8⁺ T cells and for evaluating tumor immunity as a whole. For this reason, the method of the invention can be deemed as a method that is effective against a broad range of cancer and tumor. Since the present invention is for evaluating the balance of the overall antitumor immune responses, the invention is predicted to be effective for not only immune checkpoint inhibitors against PD-1/PD-L1, but also anticancer therapy acting on other immune checkpoints. Since cancer therapy such as radiation therapy, molecularly targeted drug administration, and surgical operation treat tumor in cooperation with antitumor immunity within the body, this can also be considered effective in cancer therapy other than cancer immunotherapy through the biomarker of the invention which can evaluate the status of antitumor immunity or a method using the same.

The presence of a large quantity of a specific cell population such as a CCR4⁻CCR6⁺ cell subpopulation, CCR4⁻ CCR6⁺CXCR3⁺ cell subpopulation, or CCR4⁻CCR6⁺CXCR3⁻ cell subpopulation of the invention can be evaluated to mean that T cell immunity sufficient to exert a natural antitumor effect is prepared. Thus, the type of cancer from which a patient to be subjected to therapy is suffering is not particularly limited. Cancer may be either solid cancer or hematopoietic tumor. An effect of cancer therapy on cancer to be treated associated with antitumor immunity can be predicted in, for example, non-small cell lung cancer, small cell lung cancer, renal cancer, Hodgkin's disease, head and neck cancer, breast cancer, gastric cancer, malignant melanoma, colon cancer, acute myeloid leukemia, chronic myeloid leukemia, acute lymphatic leukemia, gastrointestinal stromal tumor, pancreatic neuroendocrine tumor, skin cancer, etc.

Although not wishing to be bound by any theory, an antitumor effect of an immune checkpoint inhibitor represented by an anti-PD-1 antibody is determined by the status of T cell immunity that is preexisting in a cancer bearing host. This is utilized to enable prediction of the presence/absence of short-term response of a PD-1 inhibitor therapy, progression free survival, and overall survival with %CD62L^{low}/CD4⁺CD3⁺ T cells obtained by cluster analysis on peripheral blood T cells in Japanese Patent No. 6664684. This performance cannot be reproduced by using CCR7, which is a homing molecule to the same secondary lymph tissue as CD62L, and nothing exceeded %CD62L^{low}/CD4⁺CD3⁺ T cells in predictive performance analysis using a T cell cluster classified into about 120 types.

Meanwhile, as described above, CD4 T cells are known to change into several differentiation states as effector cells, including Th1 which mainly produces IFNγ and is strongly associated with cellular immunity, Th2 which mainly produces IL-4 and IL-5 and is associated with allergies, Th17 which is known to be associated with autoimmune diseases, etc. and produces IL-17, etc. CD4 T cells differentiated into each state are known to express a characteristic chemokine receptor (CCR). The Th type can be classified by the type of CCR expressed. Th differentiation of human CD4 T cells is known to be more complex compared to mice, etc., and can be classified, for example, as shown in Figure **1** (Annual Review of Immunology, Vol. 34: 317-334 Heterogeneity of Human CD4+ T Cells Against Microbes, Fig. 1) .

The present invention utilizes the fact that CD62L^{low}CD4⁺CD3⁺ T cells strongly correlate with antitumor immunity to further classify CD62L^{low}CD4⁺CD3⁺ T cells into Th clusters by CCR type and analyze a Th cluster most strongly correlated with an antitumor effect to obtain a new biomarker. In one embodiment, progression free survival (PFS) after pembrolizumab therapy is used as a numerical value for evaluating an antitumor effect.

As described above, the method of the invention can evaluate the status of antitumor immunity in a subject through the amount of a specific Th cluster in CD62L^{low}CD4⁺CD3⁺ T cells. Since the immune status is associated with an effect of therapy on a subject, the method of the invention can predict the effect of cancer therapy through not only cancer immunotherapy, but also radiation therapy, molecularly targeted drug administration, and surgical operation.

In one embodiment of the invention, a CXCR3⁺ cell subpopulation can also be used in addition to a CCR4⁻CCR6⁺ cell subpopulation. Such a cell subpopulation is CCR4⁻ CCR6⁺CXCR3⁺, and classified as Th1/17 when classified by the Th type.

In one embodiment of the invention, a CXCR3⁻ cell subpopulation can also be used in addition to a CCR4⁻CCR6⁺ cell subpopulation. Such a cell subpopulation is CCR4⁻ CCR6⁺CXCR3⁻, and classified as CCR6 SP (single positive) when classified by the Th type.

In still another embodiment of the invention, a FoxP3⁻ cell subpopulation can also be used in addition to a CCR4⁻ CCR6⁺ cell subpopulation. Such a cell subpopulation is CCR4⁻CCR6⁺FoxP3⁻, and is a combination of Th1/17 and CCR6 SP when classified by the Th type.

In still another embodiment of the invention, a CD62L^{low} cell subpopulation can also be used in addition to a CCR4⁻ CCR6⁺ cell subpopulation, or a CD45RA⁻ cell subpopulation can also be used in addition to a CCR4⁻CCR6⁺ cell subpopulation.

As described above, the present invention utilizes the fact that CD62L^{low}CD4⁺CD3⁺ T cells strongly correlate with antitumor immunity to further classify CD62L^{low}CD4⁺CD3⁺ T cells into Th clusters by CCR type and analyze a Th cluster most strongly correlated with an antitumor effect to obtain a new biomarker. The inventors have performed TCR repertoire analysis using single cell RNAseq in order to study the significance of the traits of CD62L^{low}. It was found as a result that the CD62L^{low} fraction in circulating peripheral blood is comprised of few clones with very high selectivity, and only CD62L^{low} T cell clones increase after release of a cancer antigen in radiation therapy. In view of the above, the inventors have discovered that CD62L^{low} T cells are comprised of cancer antigen specific T cell clones and serve a critical role in exerting an antitumor effect in the body.

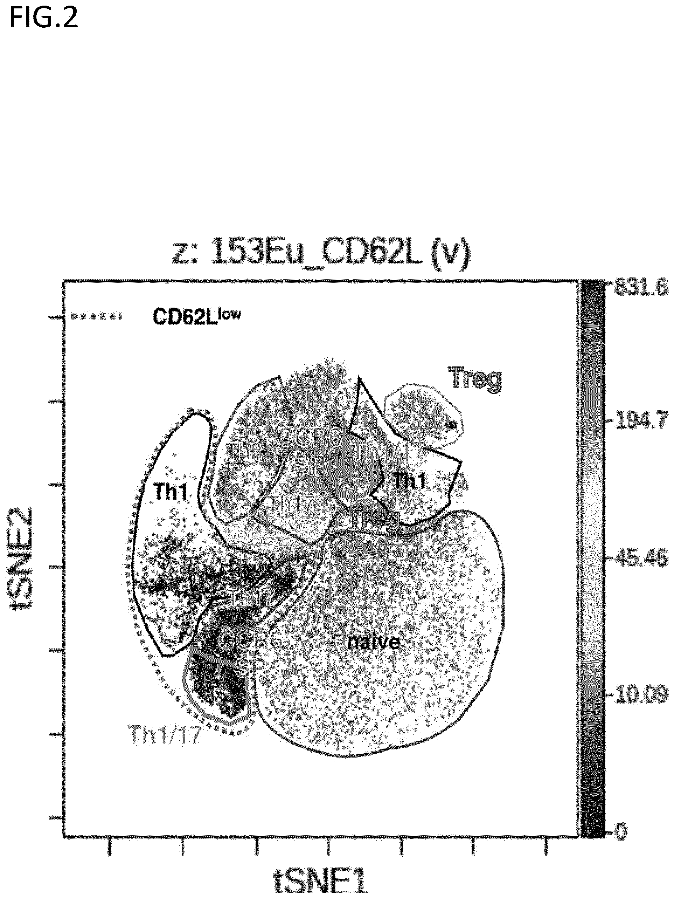

The present invention has elucidated which cluster is important for antitumor immunity among five types of Th classifications (Th1, Th1/17, Th17, CCR6 SP, and TP) contained in CD62L^{low}CD4⁺ T cells when CD4⁺ cells are mapped by viSNE analysis of CyTOF as shown in Figure **2****.** Although not wishing to be bound by any theory, since T cells which react to cancer antigen release and expand clones are found only in CD62L^{low}CD4⁺ T cells, one of five types of Th classifications contained in CD62L^{low}CD4⁺ T cells would be responsible for an antitumor effect. For this reason, one aspect of the invention can provide a method of selecting a cluster or cell subpopulation that can suitably evaluate the immune status in accordance with each cancer type or therapeutic method therefor from among five types of Th classifications contained in CD62L^{low}CD4⁺ T cells and using the amount or relative amount of the cell subpopulation as an indicator for predicting a response of a subject to cancer therapy.

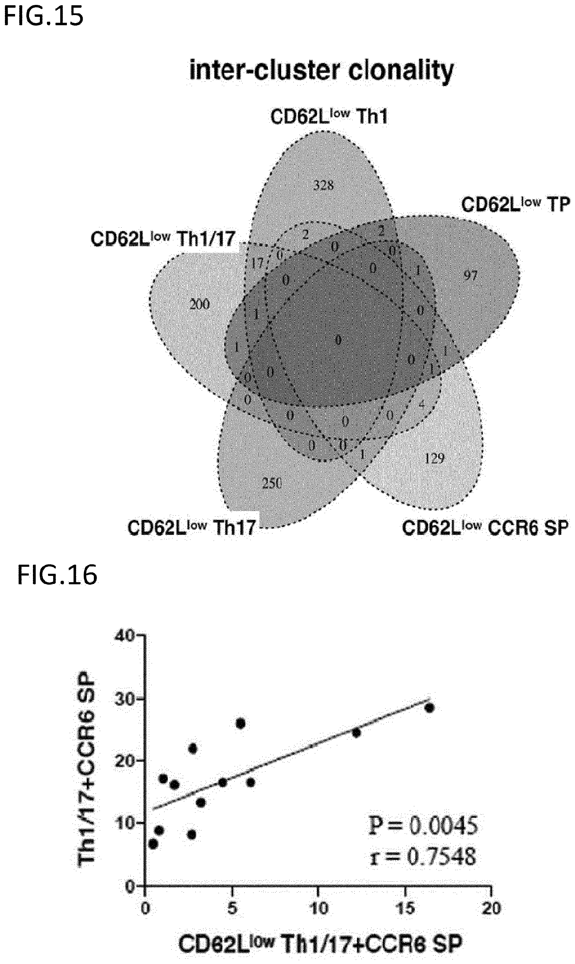

The present invention has discovered that clusters of CD62L^{low}CXCR3⁺CCR6⁺CCR4⁻ CD4⁺ and CD62L^{low}CXCR3⁻CCR6⁺CCR4⁻ CD4⁺ exhibit significant correlation with progression free survival, as shown in the Examples. Although not wishing to be bound by any theory, it is understood from viSNE analysis of CyTOF that the two clusters are adjacent and various molecule expressions are similar as shown in Figure **2****.** The clusters have been revealed to have similar gene expression patterns while having different gene expression in results using Single cell RNAseq. Meanwhile, the two clusters have different gene expression pattern from Th1 and Th17 cells (Figure **15**). Thus, it is understood that the two clusters are in differentiated state that exerts a cellular function, which is similar to each other but different from Th1 and Th17 cells. The present invention has also discovered that the ratio of CXCR3⁺CCR6⁺CCR4⁻ and CXCR3⁻CCR6⁺CCR4⁻ cells to the entire CD4⁺ cells exhibits significant correlation with the ratio of CD62L^{low}CXCR3⁺CCR6⁺CCR4⁻, CD62L^{low}CXCR3⁻CCR6⁺CCR4⁻, and CD62L^{low} CD4⁺ cells as shown in the Examples (Figure **16**), whereby the same predictive performance as the ratio of CD62L^{low}CXCR3⁺CCR6⁺CCR4⁻CD4⁺ and CD62L^{low}CXCR3⁻CCR6⁺CCR4⁻CD4⁺ cells can be achieved by using the ratio of CXCR3⁺CCR6⁺CCR4⁻ and CXCR3⁻CCR6⁺CCR4⁻ cells without using CD62L.

It is understood that the T cell composition is important in antitumor immune responses. Meanwhile, CD62L^{low}CXCR3⁺CCR6⁺CCR4⁻ CD4⁺ cells and CD62L^{low}CXCR3⁻ CCR6⁺CCR4⁻ CD4⁺ cells have a gene expression pattern that is unique among CD62L^{low}CD4⁺ T cells and have a different T cell receptor clonotype from other CD62L^{low}CD4⁺ T cells. It is understood that gene expression that is unique to CD62L^{low}CXCR3⁺CCR6⁺CCR4⁻ CD4⁺ cells and CD62L^{low}CXCR3⁻ CCR6⁺CCR4⁻ CD4⁺ cells is responsible for CD4⁺ T cell functions that are essential for helping priming, migratory capability, tumor infiltration capability, and cytotoxic function of CD8⁺ T cells via antigen presenting cells such as dendritic cells, and the unique clonotype has cancer antigen specificity. Unless CD62L^{low}CXCR3⁺CCR6⁺CCR4⁻ CD4⁺ cells and/or CD62L^{low}CXCR3⁻CCR6⁺CCR4⁻ CD4⁺ cells having such a cellular function and antigen specificity are prepared, a sufficient antitumor immune response cannot be achieved even if an immune checkpoint inhibitor is administered. For this reason, the ratio of CD62L^{low}CXCR3⁺CCR6⁺CCR4⁻ CD4⁺ cells and/or CD62L^{low}CXCR3⁻CCR6⁺CCR4⁻ CD4⁺ cells in CD4⁺ T cells would be an indicator for predicting an antitumor effect by an immune checkpoint inhibitor. The present invention has discovered, as a result of mathematical network analysis, that the ratio of CD62L^{high}CXCR3⁺CCR6⁺CCR4⁻ cells and/or CD62L^{high}CXCR3⁻CCR6⁺CCR4⁻ cells, which exhibit similar CCR expression patterns but express CD62L, to CD4⁺ T cells has a constant correlation with the ratio of CD62L^{low}CXCR3⁺CCR6⁺CCR4⁻CD4⁺ cells and/or CD62L^{low}CXCR3⁻ CCR6⁺CCR4⁻CD4⁺ cells. This result shows the amount or relative amount of a CCR4⁻CCR6⁺ cell subpopulation, which is the sum of all CXCR3⁺CCR6⁺CCR4⁻ and CXCR3⁻CCR6⁺CCR4⁻ cells in CD4⁺ cells, can be an indicator for predicting an antitumor effect from cancer therapy comprising an immune checkpoint inhibitor, while maintaining the same predictive performance.

In one aspect of the invention, the present invention can be a method of searching for or determining an indicator for predicting a response to cancer therapy (e.g., therapy using an immune checkpoint inhibitor), comprising:
*correlating a subpopulation in CD62L^{low}CD4⁺ T cells of a subject before the cancer therapy with a response of the subject to cancer therapy; and
*based on the correlation, using a subpopulation correlated with the cancer therapy as an indicator for predicting a response to the cancer therapy. In a preferred embodiment, the subpopulation can be any one of the five types of Th classification, i.e., Th1 (CXCR3⁺CCR6⁻CCR4⁻), Th1/17 (CXCR3⁺CCR6⁺CCR4⁻), Th17 (CXCR3⁻CCR6⁺CCR4⁺), CCR6 SP (CXCR3⁻ CCR6⁺CCR4⁻), and TP (CXCR3⁺CCR6⁺CCR4⁺).

Specifically, the present invention can, as described above, provide a method of selecting a cluster or cell subpopulation that can suitably evaluate an immune status in accordance with each cancer type or a therapeutic method therefor from among five types of Th classification contained in CD62L^{low}CD4⁺ T cells and using the amount or relative amount of the cell subpopulation as an indicator for predicting a response of a subject to cancer therapy. Specific examples of the cluster or cell subpopulation that can suitably evaluate an immune status in accordance with each cancer type or a therapeutic method therefor in addition to or in place of the five types of Th classification include six types of Th classification contained in CD62L^{high}CD4⁺ T cells, i.e., a CD62L^{high} Th1 (CXCR3⁺CCR4⁻CCR6⁻) cell subpopulation, CD62L^{high} Th1/17 (CXCR3⁺CCR4⁻CCR6⁺) cell subpopulation, CD62L^{high} CCR6 SP (CXCR3⁻CCR4⁻CCR6⁺) cell subpopulation, CD62L^{high} Th17 (CXCR3⁻ CCR4⁺CCR6⁺) cell subpopulation, CD62L^{high} Th2 (CXCR3⁻ CCR4⁺CCR6⁻) cell subpopulation, and CD62L^{high} TN (CXCR3⁻CCR4⁻ CCR6⁻) cell subpopulation.

Furthermore, examples of an effector CD4⁺ T cell subpopulation that can suitably evaluate an immune status by adding a Th classification subpopulation include a CCR7⁺CD62L^{low}CD4⁺ T cell subpopulation, PD1⁺CCR7⁺CD62L^{low}CD4⁺ T cell subpopulation, LAG-3⁺CCR7⁺CD62L^{low}CD4⁺ T cell subpopulation, and CD45RA⁻Foxp3⁻CD4⁺ T cell subpopulation. Examples of a regulatory T cell subpopulation include a CD127⁺CD25⁺CD4⁺ T cell subpopulation, CD45RA⁻Foxp3⁺CD4⁺ T cell subpopulation, and Foxp3⁺CD25⁺CD4⁺ T cell subpopulation. Examples of an effector CD8⁺ T cell subpopulation include a CD62L^{low}CD8⁺ T cell subpopulation.

### (Kit)

One aspect of the invention provides a kit for predicting a response of a subject to cancer therapy such as cancer immunotherapy, radiation therapy, molecularly targeted drug administration, or surgical operation, comprising a detecting agent for a cell surface marker. The inventors have discovered that these cell surface markers expressed by a T cell of a subject are related to long-term progression free survival of a subject from cancer therapy such as cancer immunotherapy, radiation therapy, molecularly targeted drug administration, or surgical operation, whereby a kit comprising a detecting agent for these cell surface markers is understood to be useful in predicting long-term progression free survival from cancer therapy such as cancer immunotherapy, radiation therapy, molecularly targeted drug administration, or surgical operation. Such a kit can comprise one or more detecting agents for a molecule suitable for detecting the cell subpopulation described herein. A combination of such detecting agents can be used for determining the T cell composition of a subject. Such a kit can be used for measuring a ratio of a specific cell subpopulation as a novel biomarker described herein in a subject.

In one embodiment of the invention, the kit of the invention can predict a response of a subject to cancer therapy such as cancer immunotherapy, radiation therapy, molecularly targeted drug administration, or surgical operation by using comparison of a relative amount of a CCR4⁻CCR6⁺ cell subpopulation in a CD4⁺ T cell population of a subject with a reference value as an indicator for predicting a response of the subject to cancer therapy such as cancer immunotherapy, radiation therapy, molecularly targeted drug administration, or surgical operation. In another embodiment, the kit of the invention can predict a response of a subject to cancer therapy such as cancer immunotherapy, radiation therapy, molecularly targeted drug administration, or surgical operation by using comparison of a relative amount of a CCR4⁻CCR6⁺ cell subpopulation in a CD4⁺ T cell population of the subject with a non-responder group threshold value as an indicator of whether the subject is a part of a non-responder group to cancer therapy such as cancer immunotherapy, radiation therapy, molecularly targeted drug administration, or surgical operation.

In one embodiment of the invention, the kit of the invention can comprise a detecting agent for CD4, CCR4, and CCR6. In another embodiment, the kit of the invention can further comprise a detecting agent for CXCR3 and CD62L. In one embodiment, a detecting agent is an antibody. Preferably, an antibody is suitably labeled and facilitates detection of a marker.

In one embodiment, the T cell composition of a subject is a composition of T cells in a sample obtained from the subject. The sample is preferably a peripheral blood sample. Since a biomarker provided in the present invention can be measured using a peripheral blood sample, this is a significant advantage in clinical applications in that the biomarker can be used noninvasively at a low cost over time.

In one embodiment, cancer immunotherapy comprises administration of an immune checkpoint inhibitor. The biomarker of the invention can accurately predict a response of a subject to especially such cancer immunotherapy.

In a preferred embodiment of the invention, additional therapy is considered to be administered when a subject is demonstrated to be unresponsive to cancer therapy such as cancer immunotherapy, radiation therapy, molecularly targeted drug administration, or surgical operation, e.g., the amount or relative amount of a CCR4⁻CCR6⁺ cell subpopulation in a CD4⁺ T cell subpopulation is less than a reference value. In such a case, therapy that is not cancer immunotherapy (e.g., chemotherapy, radiation therapy, surgical procedure, hyperthermia therapy, etc.) or more cancer immunotherapy (e.g., immune checkpoint inhibitor, adoptive cell transfer, etc.) can be administered in addition to cancer immunotherapy being administered, as an addition therapy. Typically, concomitant use of another chemotherapeutic drug or a second immune checkpoint inhibitor can be considered with an immune checkpoint inhibitor already being administered. Any therapy described herein can be administered as a therapy being combined.

In one embodiment, an immune checkpoint inhibitor comprises a PD-1 inhibitor or a PD-L1 inhibitor. Examples of a PD-1 inhibitor include, but are not limited to, an anti-PD-1 antibody that inhibits the interaction (e.g., bond) between PD-1 and PD-L1, e.g., anti-PD-1 antibodies such as nivolumab and pembrolizumab. Examples of a PD-L1 inhibitor include, but are not limited to, an anti-PD-L1 antibody that inhibits interaction (e.g., bond) between PD-1 and PD-L1, e.g., anti-PD-L1 antibodies such as durvalumab, atezolizumab, and avelumab.

Another aspect of the invention provides a method of predicting a response of a subject to cancer therapy such as cancer immunotherapy, radiation therapy, molecularly targeted drug administration, or surgical operation using the composition of T cells of the subject to treat the subject with cancer. Alternatively, a method of treating cancer in a subject with a specific composition of T cells or a composition therefor is provided. It is known that a difference in responsiveness is large for each subject in cancer immunotherapy, especially immune checkpoint inhibition therapy. Administration of cancer immunotherapy after selecting a subject with the biomarker of the invention can significantly increase the probability of achieving a therapeutic effect such as reduction of tumor.

Cancer therapy such as cancer immunotherapy, radiation therapy, molecularly targeted drug administration, or surgical operation can be administered to a subject demonstrated to be responsive to cancer therapy such as cancer immunotherapy, radiation therapy, molecularly targeted drug administration, or surgical operation by using the method described herein, including those described above. Cancer immunotherapy can be any cancer immunotherapy described herein.

Another embodiment of the invention provides a method of treating a subject with cancer, comprising: determining a relative amount of a CCR4⁻CCR6⁺ cell subpopulation in a CD4⁺ T cell population in a sample derived from the subject; determining that the subject is responsive to cancer therapy such as cancer immunotherapy, radiation therapy, molecularly targeted drug administration, or surgical operation when the relative amount of a CCR4⁻CCR6⁺ cell subpopulation in a CD4⁺ T cell population is greater than a reference value; and administering the cancer therapy such as cancer immunotherapy, radiation therapy, molecularly targeted drug administration, or surgical operation to the subject when the subject is determined to be responsive to the cancer therapy such as cancer immunotherapy, radiation therapy, molecularly targeted drug administration, or surgical operation.

Another aspect of the invention is a composition for treating cancer in a subject predicted to be responsive to cancer immunotherapy, comprising an immune checkpoint inhibitor. The present invention can also provide a product comprising a package insert and an immune checkpoint inhibitor. A package insert may describe an instruction for using an immune checkpoint inhibitor in accordance with one or more steps of the method described herein.

One embodiment of the invention is a composition for treating cancer in a subject predicted to be responsive to cancer immunotherapy, comprising an immune checkpoint inhibitor, wherein the subject has a relative amount of a CCR4⁻CCR6⁺ cell subpopulation in a CD4⁺ T cell population that is greater than or equal to a reference value.

In one embodiment, a composition comprises a PD-1 inhibitor. A PD-1 inhibitor can be, for example, an anti-PD-1 antibody that inhibits a bond between PD-1 and PD-L1 such as nivolumab or pembrolizumab. In another embodiment, a composition comprises a PD-L1 inhibitor. A PD-L1 inhibitor can be, for example, an anti-PD-L1 antibody that inhibits a bond between PD-1 and PD-L1 such as durvalumab, atezolizumab or avelumab. It is understood that such compositions comprising an immune checkpoint inhibitor achieve a therapeutic effect at a particularly high probability when administered to a subject selected with the biomarker of the invention. The composition of the invention may be concomitantly used with any other agent.

One embodiment of the invention provides a method of using the cell subpopulation composition in a subject who has undergone cancer therapy such as cancer immunotherapy, radiation therapy, molecularly targeted drug administration, or surgical operation as an indicator for predicting a response to cancer therapy such as cancer immunotherapy, radiation therapy, molecularly targeted drug administration, or surgical operation. The method can comprise analyzing the cell subpopulation composition in a sample. The cell subpopulation composition can be analyzed by any method that is described herein or known to those skilled in the art. The method may be an in vitro or in silico method. In one embodiment of the invention, comparison of the amount of a cell subpopulation with a suitable reference shows the presence/absence of immune activation in a subject. In particular, a CCR4⁻CCR6⁺ cell subpopulation in a CD4⁺ T cell population can be used as the cell subpopulation.

### (Cell therapy and expansion of cells of interest therefor)

The inventors have discovered that there is a clear correlation between the ratio of a specific cell population such as a CCR4⁻CCR6⁺ cell subpopulation, CCR4⁻CCR6⁺CXCR3⁺ cell subpopulation, or CCR4⁻CCR6⁺CXCR3⁻ cell subpopulation in a CD4⁺ T cell population and a response to cancer therapy such as cancer immunotherapy (especially cancer immunotherapy using a cancer immune checkpoint inhibitor), radiation therapy, molecularly targeted drug administration, or surgical operation, specifically, it is essential to include a large quantity of a specific cell subpopulation, which is T cells exerting an antitumor effect, in order to exert an antitumor effect with a cancer immune checkpoint inhibitor.

Although not wishing to be bound by any theory, it is understood from this finding that immunity is evaded by attenuation of antigen recognition signals due to immune checkpoint molecule expression while having T cell immunity that is sufficient to exert a naturally antitumor effect when a cancer patient has a large quantity of a specific cell population such as a CR4⁻CCR6⁺ cell subpopulation (especially a CD62L^{low}CCR4⁻CCR6⁺ cell subpopulation), CCR4⁻ CCR6⁺CXCR3⁺ cell subpopulation (especially a CD62L^{low}CCR4⁻ CCR6⁺CXCR3⁺ cell subpopulation), or CCR4⁻CCR6⁺CXCR3⁻ cell subpopulation (especially a CD62L^{low}CCR4⁻CCR6⁺CXCR3⁻ cell subpopulation) in a CD4⁺ T cell population. Meanwhile, it is understood that even if an immune evasion mechanism is blocked with an immune checkpoint inhibitor, recovery of CD4⁺ T cell function that can help the CD8⁺ T cell function cannot be expected, resulting in an unsatisfactory antitumor effect in patients who do not include a large quantity of such specific cell populations.

It is understood in view of the above that an antitumor effect can be exerted/enhanced with an immune checkpoint inhibitor by administering specific cell populations such as a CD62L^{low}CCR4⁻CCR6⁺ cell subpopulation, CD62L^{low}CCR4⁻ CCR6⁺CXCR3⁺ cell subpopulation, CD62L^{low}CCR4⁻CCR6⁺CXCR3⁻ cell subpopulation, CCR4⁻CCR6⁺ cell subpopulation, CCR4⁻ CCR6⁺CXCR3⁺ cell subpopulation, or CCR4⁻CCR6⁺CXCR3⁻ cell subpopulation in a CD4⁺ T cell population to a patient with a low amount of these specific subpopulations and thus an antitumor effect cannot be exerted with an immune checkpoint inhibitor.

Thus, another aspect of the invention is a method for improving or maintaining/sustaining a therapeutic effect of cancer immunotherapy by infusing specific cells, or a composition therefor.

It was discovered that CD62L^{low}CCR4⁻CCR6⁺CD4⁺ T cells and/or CCR4⁻CCR6⁺CD4⁺ T cells are important in a response of a subject to cancer therapy such as cancer immunotherapy, radiation therapy, molecularly targeted drug administration, or surgical operation. It is understood that responsiveness of a subject to cancer therapy such as cancer immunotherapy, radiation therapy, molecularly targeted drug administration, or surgical operation can be improved or maintained by using such T cells. One embodiment of the invention is a composition comprising CD62L^{low}CCR4⁻CCR6⁺CD4⁺ T cells or CCR4⁻CCR6⁺CD4⁺ T cells. CD62L^{low}CCR4⁻CCR6⁺CD4⁺ T cells or CCR4⁻CCR6⁺CD4⁺ T cells or a composition comprising the same are useful for concomitant use with cancer therapy such as cancer immunotherapy, radiation therapy, molecularly targeted drug administration, or surgical operation.

For example, a method of manufacturing a composition comprising CD62L^{low}CCR4⁻CCR6⁺CD4⁺ T cells or CCR4⁻CCR6⁺CD4⁺ T cells can comprise purifying CD62L^{low}CCR4⁻CCR6⁺CD4⁺ T cells or CCR4⁻CCR6⁺CD4⁺ T cells from a human derived T cell population. The purification step may comprise removing CD62L^{high} cells or CCR4⁺ cells from CCR6⁺CD4⁺ T cells (negative selection). Purification of CCR4⁻CCR6⁺CD4⁺ T cells by negative selection using an antibody and/or magnetic beads, sorting, etc. is preferred because a contaminant such as an antibody or magnetic beads would not remain on cells to be used. CD62L^{low}CCR4⁻CCR6⁺CD4⁺ T cells are obtained from positive selection by sorting CCR6⁺ cells from a cell group subjected to negative selection of CD62L^{high} cells or CCR4⁺ cells.

T lymphocytes can be retrieved in accordance with a known technology and concentrated or depleted by a known technology such as affinity binding to an antibody through immunomagnetic selection and/or flow cytometry, etc. After the concentration and/or depletion step, desired T lymphocyte in vitro expansion can be performed in accordance with a known technology (including, but not limited to, the technology described in US Patent No. 6,040,177 by Riddell, et al.) or a variation thereof that is apparent to those skilled in the art.

For example, a desired T cell population or subpopulation may be expanded by adding a first T lymphocyte population to a medium in vitro, then adding feeder cells to the medium (e.g., so that the resulting cell population comprises at least about 5, 10, 20, 40, or more feeder cells per T lymphocyte in a first population to be expanded), and incubating the culture (e.g., for a sufficient period of time to increase the T cell count). Typically, a culture can be incubated under a condition such as a temperature that is suitable for growth of T lymphocytes. For example, the temperature for growing human T lymphocytes is generally at least about 25°C, preferably at least about 30°C, and more preferably about 37°C.

Cells can be separated and/or expanded and optionally stored and then administered to a subject in accordance with a method that is described herein or a method that is well known in the art.

The amount of cells of interest (e.g., CCR4⁻CCR6⁺CD4⁺ T cells) in the composition comprising cells of the invention can be appropriately determined by those skilled in the art to achieve an intended effect, but can be, for example, at least 2 x 10⁸ cells, preferably at least 6 x 10⁸ cells, and more preferably at least 2 x 10⁹ cells.

The composition comprising cells described herein can comprise a pharmaceutically acceptable carrier or excipient in addition to cells of interest (e.g., CCR4⁻CCR6⁺CD4⁺ cell). As used herein, "pharmaceutically acceptable" means approved by a government regulatory agency, or listed in the Pharmacopoeia or other generally accepted Pharmacopoeia, for use in animals, or more specifically humans. As used herein "carrier" refers to a culture solution, infusion vehicle, irrigating solution, diluent, adjuvant, excipient, or vehicle administered in conjunction with a therapeutic agent. Since the composition comprising cells of the invention comprises cells as the primary component, a carrier that can maintain cells such as a culture solution, infusion vehicle, or irrigating solution is preferable. For example, saline and aqueous dextrose are preferred carriers when a pharmaceutical composition is intravenously administered. Preferably, a saline solution and aqueous dextrose and glycerol solution are used as a liquid carrier of an injectable solution. When a medicament is orally administered, water is a preferred carrier. Examples of suitable excipients include light anhydrous silicic acid, crystalline cellulose, mannitol, starch, glucose, lactose, sucrose, gelatin, malt, rice, wheat flour, chalk, silica gel, sodium stearate, glyceryl monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, saccharose, carboxymethylcellulose, corn starch, inorganic salt, etc. When desirable, the composition can also contain a small amount of wetting agent or emulsifier or pH buffer. These compositions can be in a form of a solution, suspension, emulsion, tablet, pill, capsule, powder, sustained release mixture, etc. It is also possible to use traditional binding agents and carriers, such as triglyceride, to prepare a composition as a suppository. Oral preparation can also comprise a standard carrier such as medicine grade mannitol, lactose, starch, magnesium stearate, sodium saccharin, cellulose, or magnesium carbonate. Examples of a suitable carrier are described in E. W. Martin, Remington's Pharmaceutical Sciences (Mark Publishing Company, Easton, U.S.A). Such a composition contains a therapeutically effective amount of therapeutic agent, preferably in a purified form, together with a suitable amount of carrier, such that the composition is provided in a form suitable for administration to a patient. A preparation must be suitable for the administration format. In addition, the preparation may additionally comprise, for example, a surfactant, excipient, coloring agent, flavoring agent, preservative, stabilizer, buffer, suspension, isotonizing agent, binding agent, disintegrant, lubricant, fluidity improving agent, corrigent, etc.

### (General technology)

The molecular biological methodology, biochemical methodology, and microbiological methodology used herein are well known and conventionally used in the art, which are described, for example, in Sambrook J. et al. (1989). Molecular Cloning: A Laboratory Manual, Cold Spring Harbor and its 3rd Ed. (2001); Ausubel, F. M. (1987). Current Protocols in Molecular Biology, Greene Pub. AssociatES and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F .M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Bessatsu Jikken Igaku [Experimental Medicine, Supplemental Volume], "Idenshi Donyu & Hatsugen Kaiseki Jikken Ho [Experimental Methods for Transgenesis & Expression Analysis]", Yodosha, 1997, etc. Relevant portions thereof (which may be the entire document) are incorporated herein by reference.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

The present invention has been described while showing preferred embodiments to facilitate understanding. While the present invention is described hereinafter based on the Examples, the above descriptions and the following Examples are provided for the sole purpose of exemplification, not limitation of the present invention. Thus, the scope of the present invention is not limited to the embodiments and Examples that are specifically described herein and is limited only by the scope of claims.

### [Example]

The present invention has been described while showing preferred embodiments to facilitate understanding. While the present invention is described hereinafter based on the Examples, the above descriptions and the following Examples are provided for the sole purpose of exemplification, not limitation of the present invention. Thus, the scope of the present invention is not limited to the embodiments and Examples that are specifically described herein and is limited only by the scope of claims.

### (Example 1: Therapeutic effect of anti-PD-1 antibody and T cell population composition)

### 1. Objective

This Example utilized the fact that CD62L^{low}CD4⁺CD3⁺ T cells are strongly correlated with antitumor immunity (Japanese Patent No. 6664684) to further classify CD62L^{low}CD4⁺CD3⁺ T cells into Th clusters by the CCR type and analyze a Th cluster that is the most strongly correlated with an antitumor effect.

### 2. Materials and Methods

Mononuclear cells were isolated and cryopreserved (liquid nitrogen) by using Vacutainer^{™} blood collection tubes from peripheral blood derived from a patient before undergoing pembrolizumab therapy. Flow cytometry and mass cytometry analysis were performed using the preserved peripheral blood mononuclear cells (PBMC). Progression free survival (PFS) after pembrolizumab therapy was used as the numerical value for evaluating an antitumor effect.

### 3. Results

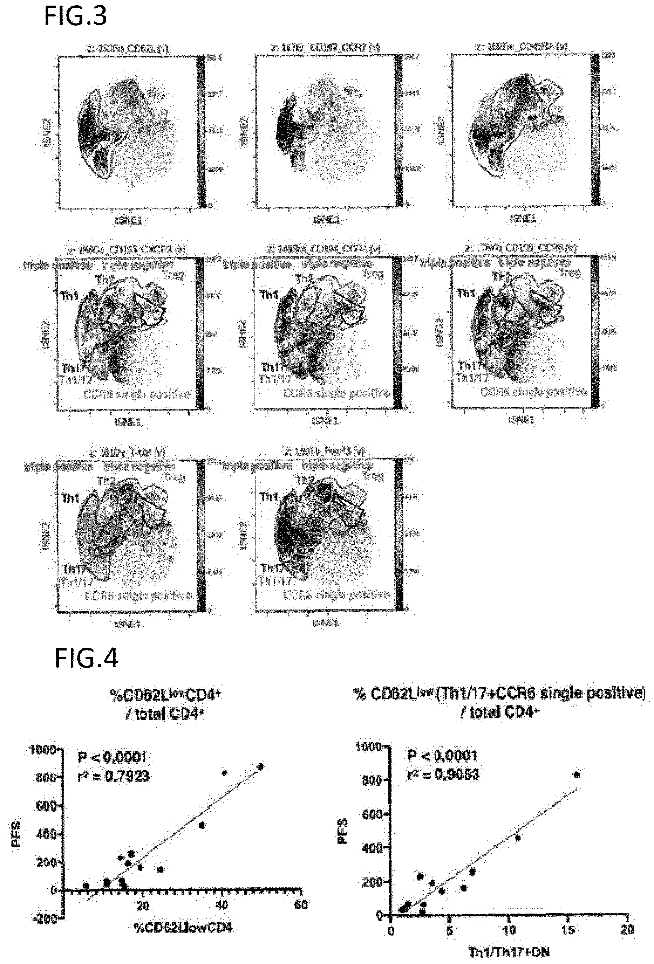

Figure **3** shows the results of mapping analysis through mass cytometry (CyTOF^{™}). The results thereof revealed that CD62L^{low} fraction and CD62L^{high}CD45RA⁻ fraction are constituted by Th clusters in the following Table 1. It is understood that the CD62L^{high}CD45RA⁺ fraction is a naive cell group without effector differentiation, and expression of CXCR3, CCR4, and CCR6 was not observed (Table 1).

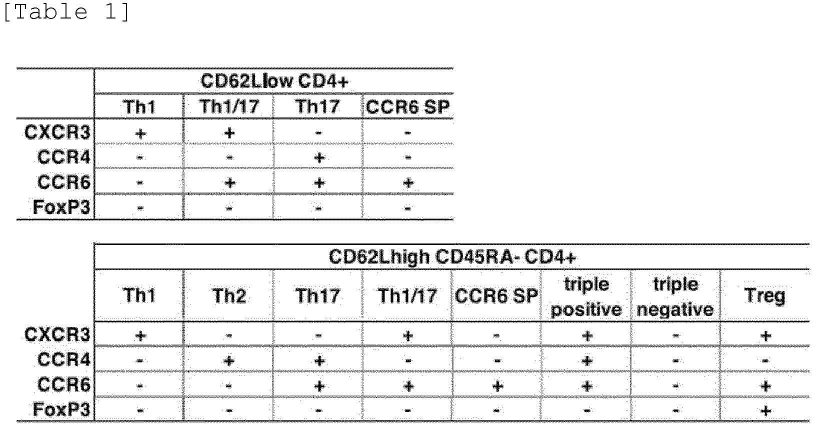

CXCR3⁺CCR6⁺CCR4⁻ (Th1/17) and CXCR3⁻CCR6⁺CCR4⁻ (CCR6 SP) exhibited significant correlation with progression free survival (PFS) among CD62L^{low} fractions (effector) (Table 2).

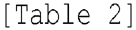

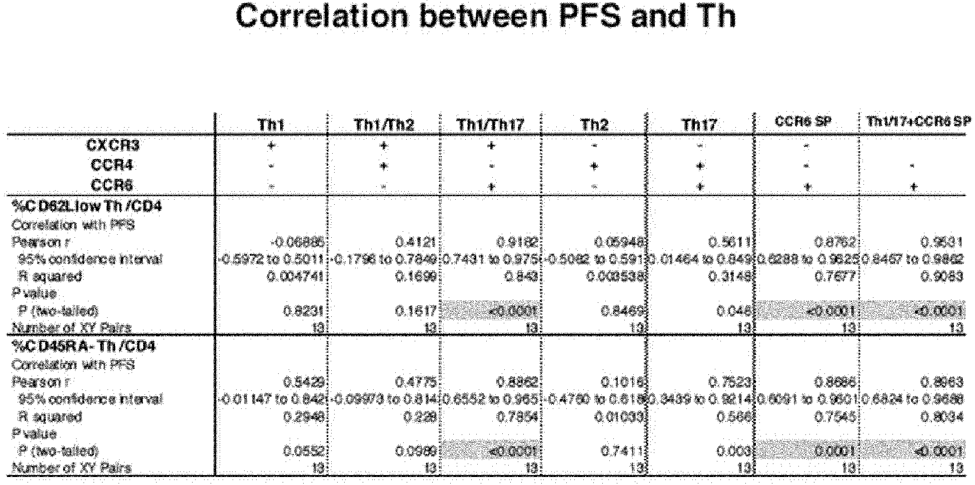

It is understood from viSNE analysis of CyTOF that the two clusters are adjacent and various molecule expressions are similar. The ratio of cells of the combination of the two clusters was very highly correlated with PFS, with P < 0.0001, r² = 0.9083 (Figure **4**) (results of 13 cases of non-small cell lung cancer as discovery cohorts). This numerical value exceeded the correlation with %CD62L^{low}/CD4⁺. Since a CD62L^{low} fraction is comprised of the Th clusters shown in Table 1, it was revealed that the following conversion is possible: %Th1/17 + %CCR6 SP = %CCR6⁺CCR4⁻.

Since clusters of the types Th1/17 and CCR6 SP are also present in the CD62L^{high}CD45RA⁻ fraction (central memory), the correlation between the ratio of said Th clusters to the entire CD45RA⁻ fraction excluding naive cells and PFS was analyzed. The Th1/17 and CCR6 SP clusters and PFS were highly correlated (Table 2). The ratio of cells of the combination of Th1/17 and CCR6 SP clusters exhibited the highest correlation.

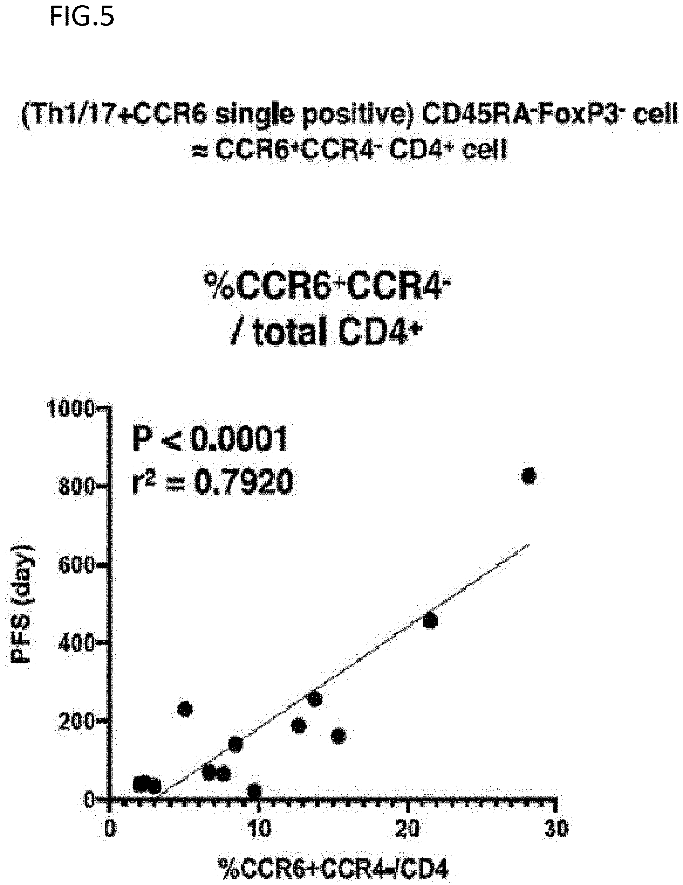

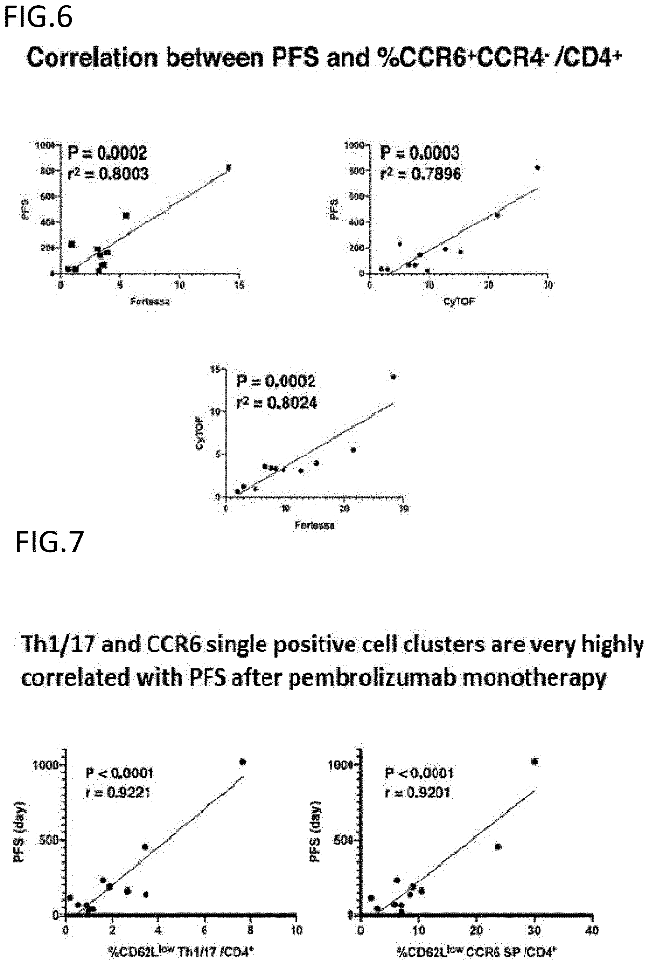

A differentiated Th cluster was not found at all in a CD62L^{high}CD45RA⁺ naive CD4 T cell fraction, and CD62L^{low} and CD62L^{high}CD45RA⁻ fractions are comprised of only Th clusters shown in Table 1. Thus, it was revealed that the ratio of cells of the combination of Th1/17 and CCR6 SP clusters to the entire CD4⁺CD3⁺ T cells is approximated by %CCR6⁺CCR4⁻ /CD4⁺. In fact, the correlation between %CCR6⁺CCR4⁻/CD4⁺ and PFS was P < 0.0001, r² = 0.7920, which is a numerical value that is inferior in terms of r² relative to %CD62L^{low}CCR6⁺CCR4⁻/CD4⁺ , but is comparable to the correlation between %CD62L^{low}/CD4⁺ and PFS (Figure **5**). %CCR6⁺CCR4⁻/CD4⁺ obtained by using common flow cytometry analysis also correlated with a numerical value obtained by CyTOF, and correlated with PFS (P = 0.0002, r² = 0.8003) (Figure **6**).

When the change after pembrolizumab therapy was analyzed, a significant increase in the ratio of Th1/17 and CCR6 SP cells was observed only in responder cases.

It was found in view of the above results that correlation with PFS was observed only in Th1/17 and CCR6 SP CD4⁺ T cell clusters as a result of PBMC analysis before pembrolizumab therapy. It was also found that %CD62L^{low} CCR6⁺CCR4⁻/CD4⁺ exhibits very high correlation with PFS exceeding that of %CD62L^{low}/CD4⁺. It was also found that %CCR6⁺CCR4⁻/CD4⁺ exhibits the same level of correlation with PFS as %CD62L^{low}/CD4⁺.

Correlation between progression free survival after pembrolizumab monotherapy and results of Th analysis through CyTOF was studied. As shown in Table 3 and Figure 7, Th1/17 and CCR6 SP CD4⁺ T cell clusters exhibited high correlation with progression free survival after pembrolizumab monotherapy.

**[Table 3]**

| Correlation between progression free survival after pembrolizumab monotherapy and results of Th analysis (CyTOF) | | | | | |
|---|---|---|---|---|---|
| vs PFS | Th1 | Th2 | Th17 | Th1/17 | CCR6 SP |
| CXCR3 | + | - | - | + | - |
| CCR4 | - | + | + | - | - |
| CCR6 | - | - | + | + | + |
| | %Th | | | CD4 | |
| Pearson r | 0.5429 | 0.1016 | 0.7523 | 0.8862 | 0.8686 |
| P value | 0.0552 | 0.7411 | 0.003 | <0.0001 | 0.0001 |
| | %CD62Llow | | | Th /CD4 | |
| Pearson r | -0.06885 | 0.05948 | 0.5611 | 0.9182 | 0.8762 |
| P value | 0.8231 | 0.8469 | 0.046 | <0.0001 | <0.0001 |

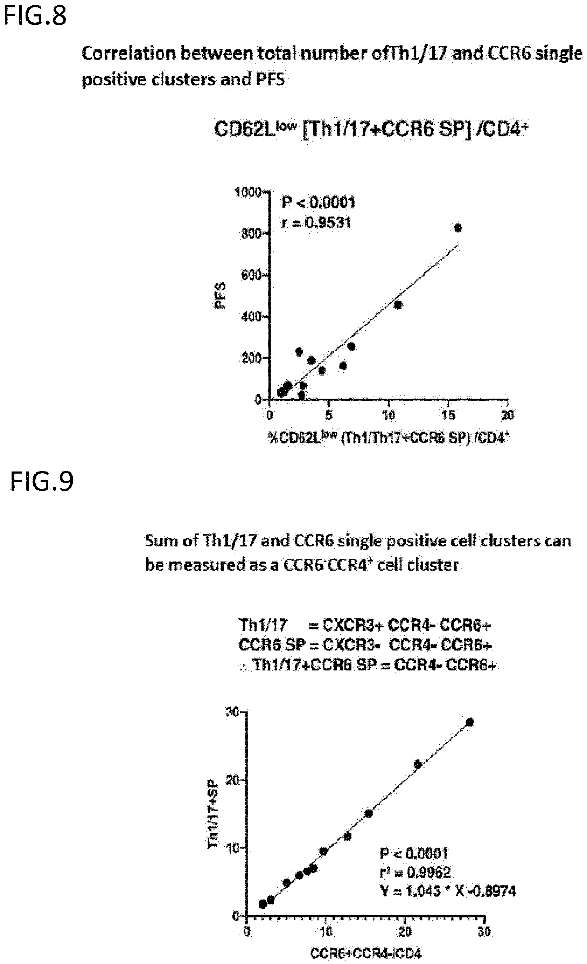

Correlation between %CD62L^{low}CD4 and %Th cluster through CyTOF was studied. As shown in the following Table 4, the ratio of Th1/17 and CCR6 SP cell clusters was correlated with a ratio of CD62L^{low} cell cluster (Figures **8** and **9**).

**[Table 4]**

| **%CD62L^{low} CD4 and %Th cluster (CyTOF)** | | | | | |
|---|---|---|---|---|---|
| vs %CD62Ltow/CD4 | Th1 | Th2 | Th17 | Th1/17 | CCR6 SP |
| CXCR3 | + | - | - | + | - |
| CCR4 | - | + | + | - | - |
| CCR6 | - | - | + | + | + |
| | %Th /CD4 | | | 5RA-CD4 | |
| Pearson r | 0.08471 | -0.6402 | 0.3607 | 0.7363 | 0.6893 |
| P value | 0.7832 | 0.0184 | 0.2259 | 0.0041 | 0.0092 |

| | | | | | |
|---|---|---|---|---|---|
| The ratio of Th1/17 and CCR6 single positive cell clusters is correlated with the ratio of CD62L^{low} cell cluster | | | | | |

### 4. Significance of Results

This Example discovered that measurement of the ratio of CCR6⁺CCR4⁻ cells to the entire CD4 T cells can achieve the same predictive performance as the ratio of CD62L^{low} CD4⁺ cells more simply than using CD62L. Analysis can also be performed globally using a simple sample, which is just fixated without Ficoll separation, as the blood sample.

### 5. Discussion

While it has been reported that T-bet⁺ Th1 type (CXCR3⁺) CD4 T cells are critical in antitumor immunity, the present invention has elucidated the importance of the Th fraction that was not previously defined, i.e., CCR6⁺CCR4⁻. Since CXCR3⁺ clusters in the CCR6⁺CCR4⁻ fraction express T-bet, the importance of a T-bet⁺ fraction is not denied, but it is a significant discovery that a classical Th1 fraction, i.e., CXCR3⁺CCR4⁻CCR6⁻, is not associated with an antitumor effect.

A CCR6⁺CCR4⁻ fraction contains both CCR7 negative and positive cells. Thus, this cluster could not be identified when analyzed using only CCR7 and CD45RA, which are used often in classification of human T cells.

Even if an approach of further studying CD62L cell fractions just like the inventors using a mouse is employed, mouse T cells differentiate into each of Th1, Th2, and Th17 and hardly have a Th1/17 fraction. Thus, the importance of a CCR6⁺CCR4⁻ fraction could not be analyzed.

In the present invention, analysis of a human CD62L^{low}CD4 T cell cluster in further detail is the basis of the discovery of the importance of a CCR6⁺CCR4⁻ CD4 T cell cluster in antitumor immunity.

### (Example 2: Correlation between Th cluster and overall survival)

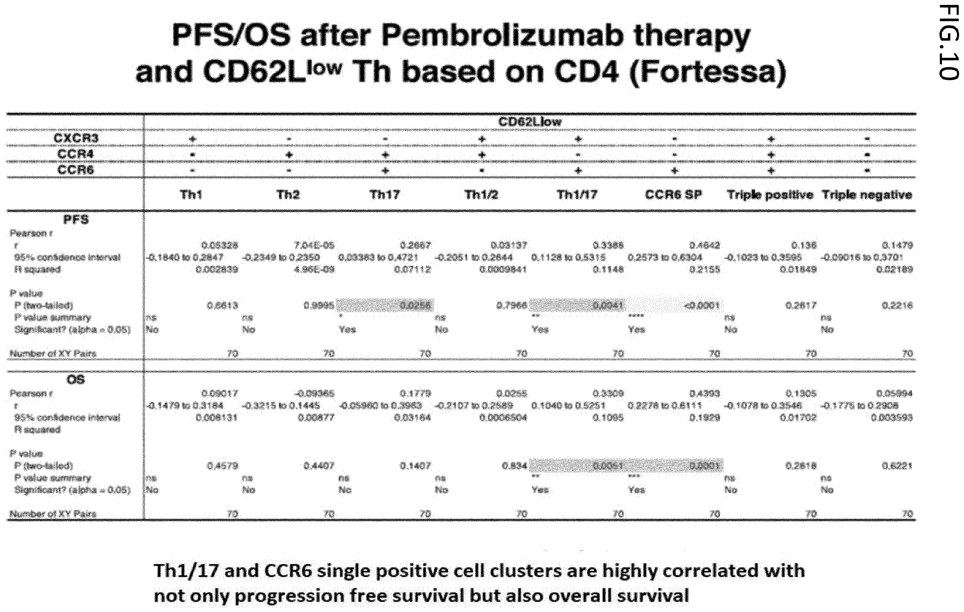

It was found that Th1/17 and CCR6 SP CD4⁺ T cell clusters among Th clusters exhibit high correlation with progression free survival after pembrolizumab monotherapy, as shown in Example 1. In this regard, the correlation between a Th cluster and overall survival was studied. The results are shown in Figure **10****.** As shown in Figure **10****,** it was found that Th1/17 and CCR6 SP CD4⁺ T cell clusters are highly correlated with not only progression free survival but also overall survival.

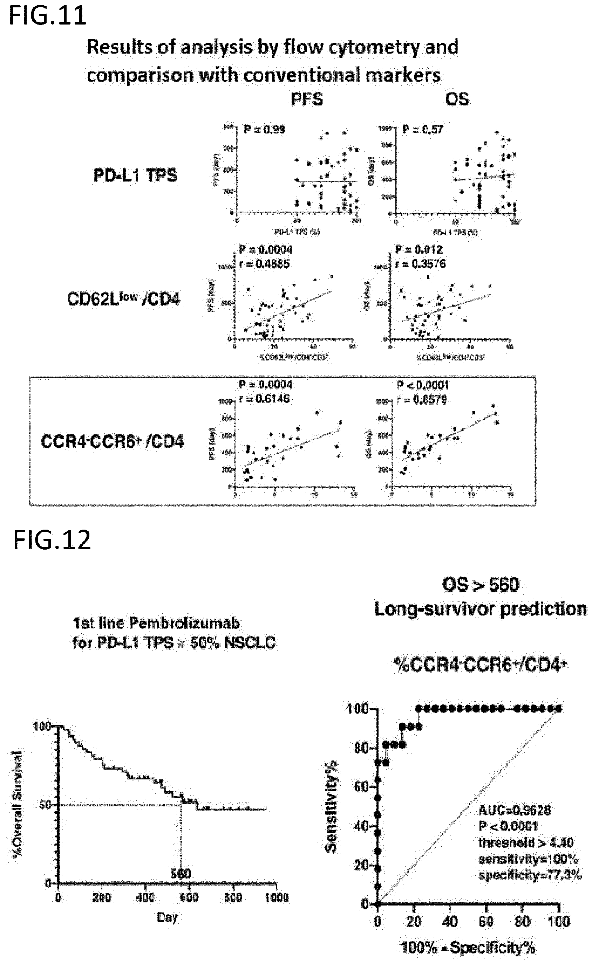

It was found as a result of analysis using flow cytometry in 35 cases of lung cancer that a CCR6⁺CCR4⁻CD4 T cell cluster exhibits higher correlation with progression free survival and overall survival relative to other conventional markers, as shown in Figure **11****.**

### (Example 3: Evaluation by sensitivity and specificity)

The performance to predict long-term survival cases for a CCR6⁺CCR4⁻CD4 T cell cluster was evaluated by ROC analysis. The results of sensitivity and specificity thereof are shown in Figure **12****.** It was found from using the ratio of CCR6⁺CCR4⁻CD4⁺ that it is a very good biomarker at a threshold value of 4.4, with sensitivity of 100% and specificity of 77.3% (Figure **12**).

### (Example 4: PFS after chemoradiotherapy on locally advanced non-small cell lung cancer)

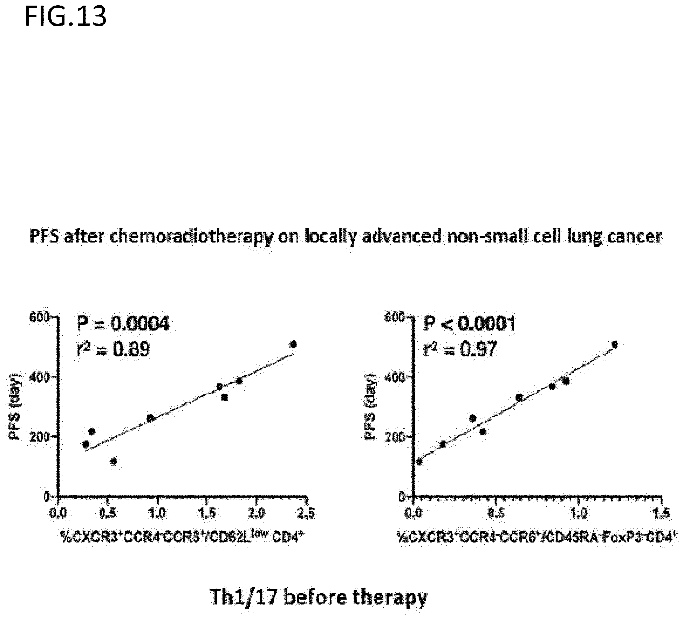

Whether PFS after chemoradiotherapy can be predicted by the amount of a CCR6⁺CCR4⁻ CD4 T cell cluster in locally advanced non-small cell lung cancer patients was subsequently studied. Figure **13** shows the results of correlation between the amount of a Th1/17 (CCR6⁺CCR4⁻ CXCR3⁺) fraction before therapy and PFS. It was found that the amount of CCR6⁺CCR4⁻ CD4 T cells is very highly correlated with PFS after chemoradiotherapy, as shown in Figure **13****.** It was found in view of the above that a CCR6⁺CCR4⁻ CD4 T cell cluster broadly reflects the immune status of a subject, so that not only the prognosis of therapy using an immune checkpoint inhibitor, but also prognosis after radiation therapy can be predicted.

### (Example 5: Prediction of effect of EGFR-TKI)

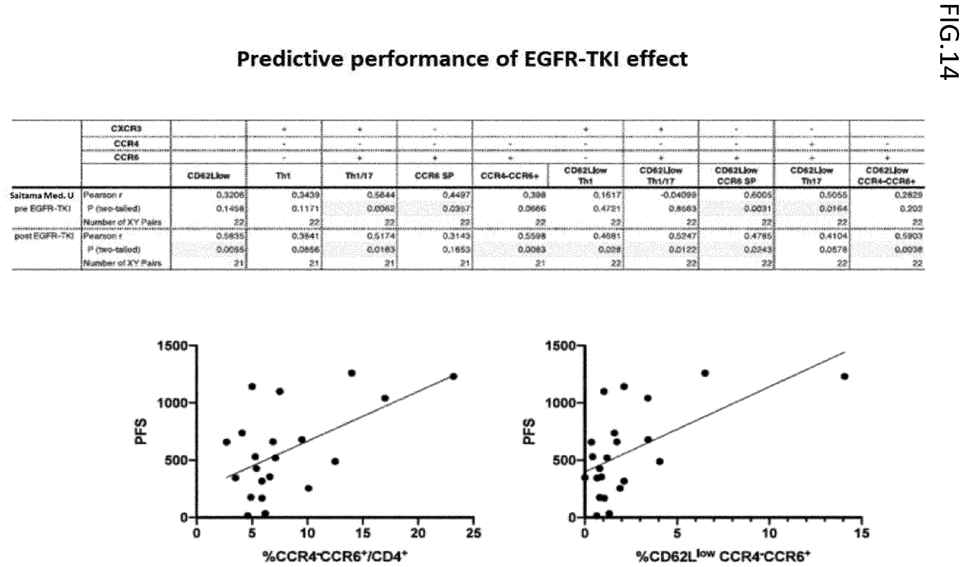

Whether PFS after EGFR-TKI therapy can be predicted by the amount of a CCR6⁺CCR4⁻ CD4 T cell cluster in locally advanced non-small cell lung cancer patients was subsequently studied. Figure **14** shows the results of correlation between the amount of a CCR6⁺CCR4⁻ fraction before therapy and PFS. It was found that the amount of CCR6⁺CCR4⁻ CD4 T cells is very highly correlated with PFS after EGFR-TKI therapy, as shown in Figure **14****.** It was found in view of the above that a CCR6⁺CCR4⁻ CD4 T cell cluster broadly reflects the immune status of a subject, so that not only the prognosis of therapy using an immune checkpoint inhibitor, but also prognosis after therapy with a molecularly targeted therapeutic agent can be predicted. Since a molecularly targeted therapeutic agent controls tumor in cooperation with immunity, prognosis can be predicted by evaluating the immune status by a CCR6⁺CCR4⁻ CD4 T cell cluster.

### (Example 6: Cell therapy for improving or maintaining/sustaining a therapeutic effect of cancer immunotherapy)

CD62L^{low}CCR6⁺CCR4⁻CD4⁺ T cells are purified from a peripheral blood sample of a subject and cryopreserved under liquid nitrogen prior to starting therapy through cancer immunotherapy. The cryopreserved CD62L^{low}CCR6⁺CCR4⁻ CD4⁺ T cells are thawed, subjected to pseudo-antigen stimulation ex vivo, then cultured in the presence of IL2 and expanded. Dendritic cells obtained by culturing peripheral blood CD16⁺ cells of the same patient in the presence of GMCSF and IL4 and patient cancer cells irradiated with radiation are co-cultured to uptake cancer antigen. CD62L^{low}CCR6⁺CCR4⁻CD4⁺ T cells expanded ex vivo with the dendritic cells that have taken up cancer antigens are co-cultured to evaluate cancer antigen specific cytokine production.

In order to maintain the effect of cancer immunotherapy, the CD62L^{low}CCR6⁺CCR4⁻CD4⁺ T cells expanded ex vivo are intravenously administered to the patient if the effect is insufficient or attenuated.

If a subject is determined to be responsive to cancer immunotherapy, cancer immunotherapy such as therapy using an anti-PD-1 antibody such as nivolumab is administered when, for example, the ratio of CCR6⁺CCR4⁻CD4⁺ T cells is high. The CD4⁺ T cell composition of the subject is monitored during therapy.

In this regard, if an indicator such as the ratio of CCR6⁺CCR4⁻CD4⁺ T cells decreases in the CD4+ T cell composition of the subject to reach an unresponsive immune status, CCR6⁺CCR4⁻CD4⁺ T cells expanded ex vivo are infused to revert back to the original immune state and sustain the effect of cancer immunotherapy.

The preservation/culture cost can be minimized by culturing and infusing only CCR6⁺CCR4⁻CD4⁺ T cells. This is more economical than continuation of only an immune checkpoint inhibitor such as an anti-PD-1 antibody every two weeks.

CCR6⁺CCR4⁻CD4⁺ T cells isolated from a subject and expanded ex vivo are infused into a subject, who has a low indicator such as the ratio of CCR6⁺CCR4⁻CD4⁺ T cells in the CD4⁺ T cell composition of the subject and determined to be unresponsive, to change the immune status to responsive, and then cancer immunotherapy using an anti-PD-1 antibody such as nivolumab is administered, whereby a subject who could not benefit from cancer immunotherapy using an anti-PD-1 antibody in the past can have an antitumor immune response in cancer immunotherapy.

### (Example 7: Correlation of Th1/17 and CCR6 SP with PFS)

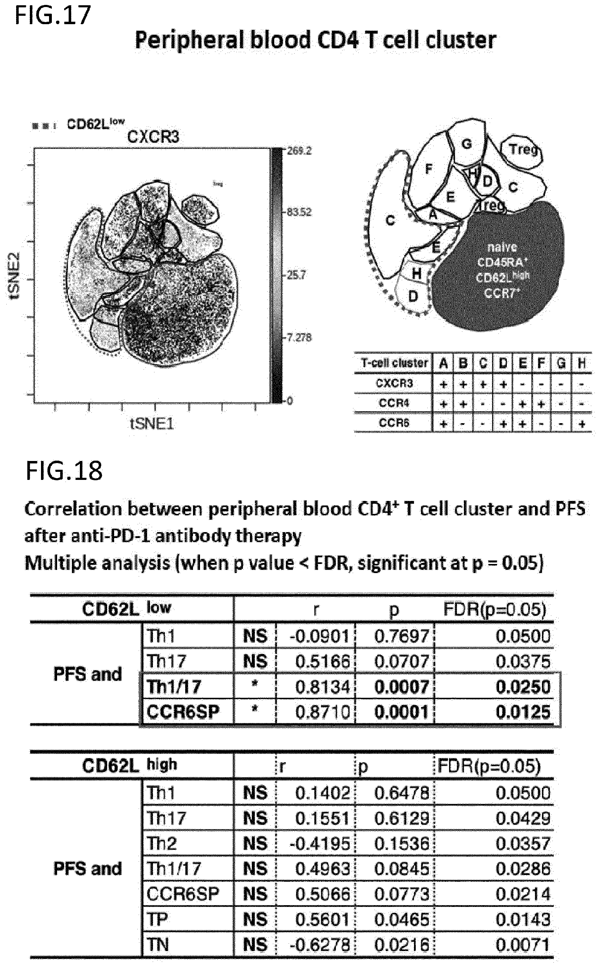

It was found that Th1/17 and CCR6 SP CD4⁺ T cell clusters among Th clusters exhibit high correlation with progression free survival as shown in Example 1. In this regard, Figure **17** schematically depicts Figure **2** and summarizes chemokine receptors expressed in each fraction in a table. As is apparent from this diagram, the portion surrounded by a dotted line is a CD62L^{low} cell population. It can be understood that there are four clusters (C, D, E, and H) with different expression of a chemokine receptor therein. Fraction D therein is Th1/17 expressed as CXCR3⁺CCR4⁻CCR6⁺, and fraction H is CCR6 SP expressed as CXCR3⁻CCR4⁻CCR6⁺.

It is known, as described above, that Th1/17 and CCR6 SP are important, from checking the correlation with progression free survival to study which of the four clusters is associated with an antitumor effect. Figure **18** shows the result of checking. Figure **18** lists correlation of PFS after anti-PD-1 antibody therapy with a peripheral blood CD4⁺ T cell cluster based on chemokine receptor expression. The correlation of all CD4⁺ T cell clusters with an effect of PD-1 inhibitor therapy was analyzed. To avoid multiplicity, FDR was 0.05 and p-value that is smaller was considered significant. As can be understood from the diagram, Th1 and Th17 cluster cell counts did not significantly correlate with PFS or OS. In a CD62L^{low} cell population, two CD4⁺ T cell clusters, i.e., Th1/17 and CCR6 SP T cell clusters, exhibited a significant positive correlation with PFS and OS.

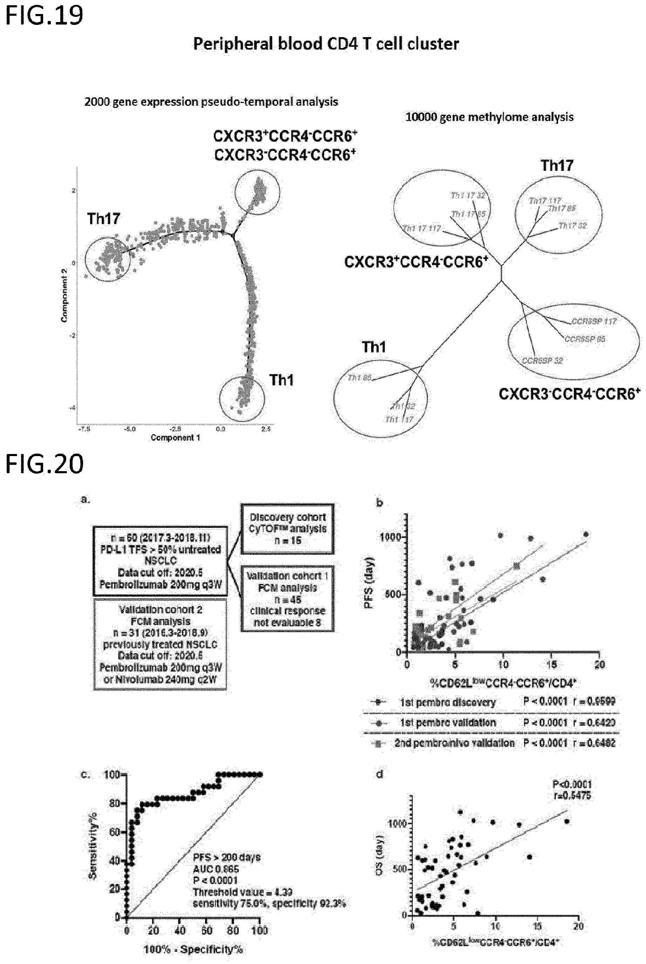

Figure **19** is a result of gene expression analysis and methylome (methylation) analysis on two peripheral blood CD4⁺ T cell clusters, i.e., Th1/17 and CCR6 SP. To study the similarity in gene expression, pseudo-temporal analysis was conducted using gene expression of the four Th clusters seen in a CD62L^{low}CD4⁺ fraction (left side of Figure **19**). As a result, the CXCR3⁺CCR4⁻CCR6⁺ (Th1/17) cluster and CXCR3⁻ CCR4⁻CCR6⁺ (CCR6 SP) cluster formed another node at the center of the position between Th1 and Th17 when Th1 and Th17 were placed at positions that were pseudo-temporally furthest away. This shows that the two clusters may be independent clusters with a property between Th1 and Th17 from the viewpoint of a gene expression profile. It was also found from hierarchically clustering gene expression profiles of six patients that the Euclidean distance between CXCR3⁺CCR4⁻CCR6⁺ (Th1/17) and CXCR3⁻CCR4⁻CCR6⁺ (CCR6 SP) is the closest. The two major factors of regulation of gene expression of T cells are activation status affected by TCR signals and differentiation status mainly due to DNA methylation. To investigate whether a difference in gene expression pattern observed in pseudo-temporal analysis corresponds to the differentiation status, methylome analysis was conducted using four T cell clusters selected from CD62L^{low}CD4⁺ T cells (right side of Figure **19**). In view of clustering analysis based on the results of the methylome analysis, Th1 and Th17 clusters were apart, and there was a CXCR3⁺CCR4⁻CCR6⁺ (Th1/17) cluster aside from these clusters, which matched the results of pseudo-temporal analysis (right side of Figure **19**). The CXCR3⁻ CCR4⁻CCR6⁺ (CCR6 SP) cluster was located between the Th17 cluster and CXCR3⁺CCR4⁻CCR6⁺ cluster.

It is known as described above that Th1/17 and CCR6 SP are correlated with progression free survival as a result of 13 cases of non-small cell lung cancer as discovery cohorts (Figure **4**). The PFS predictive performance of two peripheral blood CD4⁺ T cell clusters Th1/17 and CCR6 SP was further studied using primary treatment and treated cases of non-small cell lung cancer as validation cohorts.

A CD62L^{low} CCR4⁻CCR6⁺ CD4⁺ T cell metacluster, which is the sum of Th1/17 (CXCR3⁺CCR4⁻CCR6⁺) and CCR6 SP (CXCR3⁻ CCR4⁻CCR6⁺) T cell clusters, exhibited a strong correlation with PFS in validation cohorts (Figures 20a and b, P < 0.0001, r = 0.9599). Furthermore, the correlation of Th1/17 and CCR6 SP with PFS was investigated when pembrolizumab was administered to non-small cell lung cancer patients who have not been treated and when pembrolizumab or nivolumab was administered to non-small cell lung cancer patients who have already been treated in order to check whether a change in the correlation of Th1/17 and CCR6 SP with PFS is observed depending on the timing of therapy using an immune checkpoint inhibitor. Specifically, whether Th1/17 and CCR6 SP T cell clusters and a CD62L^{low} CCR4⁻CCR6⁺ CD4⁺ T cell metacluster correlate with the clinical outcome after immune checkpoint inhibition therapy was studied by using two validation cohorts of when pembrolizumab was administered to non-small cell lung cancer patients who have not been treated and when pembrolizumab or nivolumab was administered to non-small cell lung cancer patients who have already been treated. The two validation cohorts were NSCLC patient initially administered with pembrolizumab with PD-L1 TPS of 50% or greater and treated patient administered with pembrolizumab or nivolumab regardless of PD-L1 expression of tumor. A regression line indicating the correlation between a CD62L^{low} CCR4^{~}CCR6⁺ CD4⁺ T cell metacluster and PFS obtained in the two validation cohorts did not exhibit a significant difference from those observed in a discovery cohort in the γ-intercept or the slope. A significant correlation of Th1 and Th17 with PFS and OS was not observed in a group who received primary treatment with pembrolizumab. However, CD62L^{low} CXCR3⁺CCR4⁻ CCR6⁺ and CXCR3⁻CCR4⁻CCR6⁺ CD4⁺ T cell clusters exhibited a significant correlation. A CD62L^{low} CCR4⁻CCR6⁺ CD4⁺ T cell metacluster was increased significantly in peripheral blood before therapy compared to an advanced disease group, but there was no difference in Th1 and Th17. A CD62L^{low} CCR4⁻ CCR6⁺CD4⁺ T cell metacluster significantly predicted PFS of 200 days or longer, with an area under the curve (AUC) of 0.865 in ROC curve analysis (Figure 20c). A CD62L^{low} CCR4⁻ CCR6⁺ CD4⁺ T cell metacluster was significantly correlated with not only PFS, but also OS (Figure 20d). Since an antitumor effect of PD-1 inhibition therapy is dependent on a CD62L^{low} CCR4⁻CCR6⁺ CD4⁺ T cell metacluster, it is understood that a CD62L^{low} Th1/17 T cell cluster and CD62L^{low} CCR6 SP T cell cluster cooperate to mediate antitumor immunity. While correlation with PFS was also observed in the entire CD62L^{low} cell population including clusters other than Th1/17 and CCR6 SP, a comparatively higher correlation with PFS was able to be achieved when only Th1/17 and CCR6 SP were extracted (not shown). It is understood that this is because the entire CD62L^{low} cell population, especially when only Th1/17 and CCR6 SP, includes only clusters associated with an antitumor effect, so that the correlation with PFS was high.

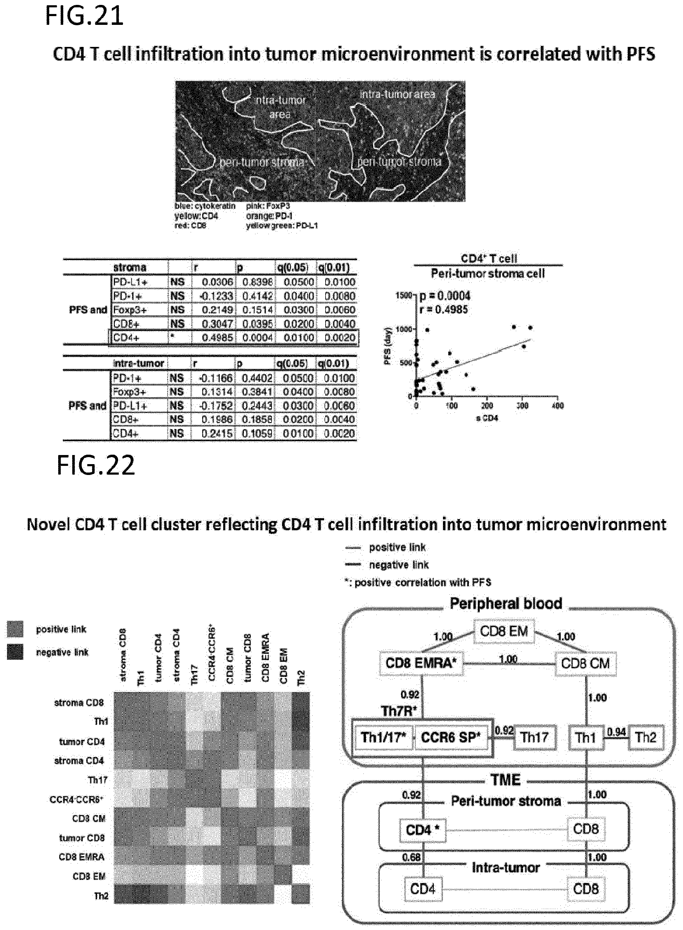

The correlation of CD4 T cells in a tumor microenvironment instead of peripheral blood with PFS was then studied. Tumor specimens (n = 46) obtained by core needle biopsy, transbronchial biopsy, or surgical biopsy were stained with cytokeratin, CD4, CD8, PD-1, PD-L1, and FoxP3. 10000 cells in tumor and the stroma around the tumor were automatically counted by using a multicolor analyzer (OPAL^{™}) based on detection of cytokeratin + tumor cells. The results are shown in Figure **21****.** The picture shown on the top part of Figure **21** is a picture of each molecule stained to investigate how much molecules such as CD4, CD8, and PD-1 are present within the tumor and stroma. When T cell infiltration into the tumor microenvironment (TME) was studied, a significant correlation of the number of CD8⁺ cells and CD4⁺ cells that are present in the stroma around tumor with PFS and OS was found in repeated correlation analysis on immune cell count in the tumor microenvironment, but the most significant correlation was observed in CD4⁺ T cells that are present in the stroma around tumor in multiple comparison analysis (bottom left and bottom right of Figure **21**).

In order to elucidate the relationship between a CD4⁺ T cell cluster found in peripheral blood correlated with an antitumor effect of a PD-1 inhibitor and the presence of T cells in a tumor microenvironment or other peripheral blood T cell clusters, multiple comparison analysis and network analysis were performed, which can investigate the relationship with which the number of each of the cell clusters in peripheral blood and tumor microenvironment (TME) is linked. Figure **22** shows results of network analysis on a T cell cluster in peripheral blood and immune cells in TME. In the Figure, the number of certain cells which changes in complete sync is indicated as 1.00, and the number becomes smaller as the link weakens. As can be understood from this figure, the results of network analysis for the combined cell count for Th1/17 and CCR6 SP in peripheral blood and the cell count for CD4⁺ cells in tumor microenvironment (TME) are indicated as 0.92, so that clusters of the combination of Th1/17 and CCR6 SP in peripheral blood are strongly associated with infiltration of CD4⁺ cells into the tumor microenvironment. Meanwhile, Th1 cells were directly associated with CD8⁺ T cells in a tumor microenvironment. In this manner, a cluster of the combination of Th1/17 and CCR6 SP in peripheral blood promoted infiltration of CD4⁺ T cells into a tumor microenvironment and CD62L^{low} Th1 cells promoted infiltration of CD8⁺ T cells. The results of network analysis revealed that a cluster of the combination of Th1/17 and CCR6 SP in peripheral blood is positively correlated with the frequency of CD45RA⁺CCR7⁻ effector CD8⁺ T cells (TEMRA) . It was found therefrom that the state of CD4⁺ cells in tumor can be understood by checking Th1/17 and CCR6 SP in peripheral blood.

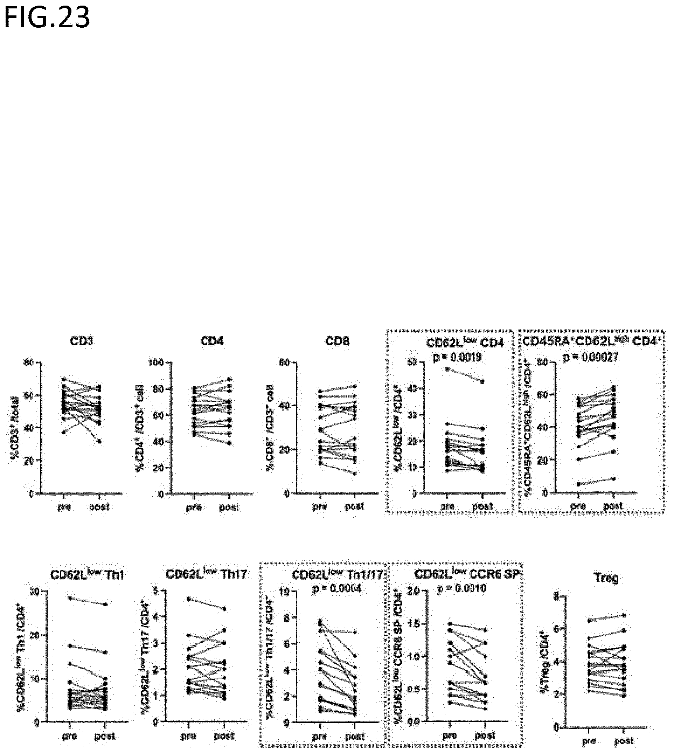

Subsequently, a cell cluster in peripheral blood before surgery was compared with a cell cluster in peripheral blood after removing tumor tissue by surgery. The results are shown in Figure **23****.** When tumor tissue is eliminated by surgery, a cell cluster that is also decreased in peripheral blood can be understood to be a cell cluster that increased due to the presence of tumor. As shown in Figure **23****,** the CD62L^{low} cell count in peripheral blood is decreased post-surgery (post) compared to pre-surgery (pre). It was found that the cell count of CD62L^{low} cells is decreased significantly post-surgery for Th1/17 and CCR6 SP in particular. It can be understood therefrom that Th1/17 and CCR6 SP are cell clusters that are associated with tumor.

As described above, the present disclosure is exemplified by the use of its preferred embodiments. It is understood that the scope of the present disclosure should be interpreted solely based on the claims. It is understood that any patent, any patent application, and any references cited herein should be incorporated herein by reference in the same manner as the contents are specifically described herein. The present application claims priority to Japanese Patent Application No. 2020-150620 filed with the JPO on September 8, 2020. The entire content thereof is incorporated herein by reference in the same manner as the contents are specifically described herein.

### [Industrial Applicability]

The biomarkers provided in the present invention can be utilized in predicting a response to a cancer immune checkpoint inhibitor in a simple, cost effective, and accurate manner, and are medically and socially essential. It is understood that the present invention is a technology needed globally for all cancer types and has a very significant market value.

## Claims

1. A method of using a relative amount of a CCR4⁻CCR6⁺ cell subpopulation in a CD4⁺ T cell population of a subject as an indicator for predicting a response of the subject to cancer therapy, comprising
determining the relative amount of the cell subpopulation in a sample derived from the subject,
wherein the relative amount is used as an indicator for predicting a response of the subject to cancer therapy.

2. The method of claim 1, wherein comparison of the relative amount with a reference value is used as an indicator for predicting a response of the subject to cancer therapy.

3. The method of claim 1 or 2, wherein comparison of the relative amount with a reference value is used as an indicator for predicting long-term survival of the subject.

4. The method of any one of claims 1 to 3, wherein comparison of the relative amount with a non-responder group threshold value is used as an indicator for predicting whether the subject is a part of a non-responder group to the cancer therapy.

5. The method of any one of claims 1 to 4, wherein the CCR4⁻CCR6⁺ cell subpopulation is also a CXCR3⁺ cell subpopulation.

6. The method of any one of claims 1 to 4, wherein the CCR4⁻CCR6⁺ cell subpopulation is also a CXCR3⁻ cell subpopulation.

7. The method of any one of claims 1 to 6, wherein the CCR4⁻CCR6⁺ cell subpopulation is also a FoxP3⁻ cell subpopulation.

8. The method of any one of claims 1 to 7, wherein the CCR4⁻CCR6⁺ cell subpopulation is also a CD62L^{low} cell subpopulation.

9. The method of any one of claims 1 to 7, wherein the CCR4⁻CCR6⁺ cell subpopulation is also a CD45RA⁻ cell subpopulation.

10. The method of any one of claims 1 to 9, wherein the cancer therapy comprises cancer immunotherapy, radiation therapy, molecularly targeted drug therapy, surgical operation, cell infusion, or any combination thereof.

11. The method of any one of claims 1 to 10, wherein the cancer therapy is cancer immunotherapy.

12. The method of claim 11, wherein the cancer immunotherapy comprises administration of an immune checkpoint inhibitor.

13. The method of claim 12, wherein the immune checkpoint inhibitor comprises an inhibitor selected from the group consisting of a PD-1 inhibitor, a PD-L1 inhibitor, and a CTLA-4 inhibitor.

14. The method of claim 12, wherein the immune checkpoint inhibitor comprises a combination of a PD-1 inhibitor or PD-L1 inhibitor and a CTLA-4 inhibitor.

15. The method of claim 13 or 14, wherein the PD-1 inhibitor is an anti-PD-1 antibody that inhibits an interaction between PD-1 and PD-L1.

16. The method of any one of claims 13 to 15, wherein the PD-1 inhibitor comprises nivolumab or pembrolizumab.

17. The method of any one of claims 13 to 16, wherein the PD-L1 inhibitor is an anti-PD-L1 antibody that inhibits an interaction between PD-1 and PD-L1.

18. The method of claim 17, wherein the PD-L1 inhibitor comprises durvalumab, atezolizumab, and avelumab.

19. The method of any one of claims 13 to 18, wherein the CTLA-4 inhibitor comprises ipilimumab and tremelimumab.

20. The method of any one of claims 4 to 19, wherein the non-responder group threshold value is determined by considering sensitivity and specificity for detection of a non-responder group.

21. The method of any one of claims 4 to 20, wherein the non-responder group threshold value is determined so that sensitivity for detection of a non-responder group exceeds about 90%.

22. The method of any one of claims 4 to 21, wherein the non-responder group threshold value is determined so that specificity for detection of a non-responder group exceeds about 90%.

23. The method of any one of claims 1 to 22, wherein a relative amount of the cells is measured using a peripheral blood sample.

24. The method of any one of claims 2 to 23, wherein the relative amount being greater than or equal to the reference value indicates that the subject is responsive to the cancer therapy.

25. A composition for treating cancer in a subject, comprising an immune checkpoint inhibitor, wherein the subject is a subject predicted to be responsive to cancer immunotherapy by the method of any one of claims 11 to 24.

26. The composition of claim 25, wherein the immune checkpoint inhibitor comprises an inhibitor selected from the group consisting of a PD-1 inhibitor, a PD-L1 inhibitor, and a CTLA-4 inhibitor.

27. The composition of claim 26, wherein the immune checkpoint inhibitor comprises a combination of a PD-1 inhibitor or PD-L1 inhibitor and a CTLA-4 inhibitor.

28. The composition of claim 26 or 27, wherein the PD-1 inhibitor is an anti-PD-1 antibody that inhibits an interaction between PD-1 and PD-L1.

29. The composition of any one of claims 26 to 28, wherein the PD-1 inhibitor comprises nivolumab or pembrolizumab.

30. The composition of any one of claims 26 to 27, wherein the PD-L1 inhibitor is an anti-PD-L1 antibody that inhibits an interaction between PD-1 and PD-L1.

31. The composition of claim 30, wherein the PD-L1 inhibitor comprises durvalumab, atezolizumab, or avelumab.

32. The composition of any one of claims 26 to 31, wherein the CTLA-4 inhibitor comprises ipilimumab.

33. A kit for predicting a response of a subject to cancer therapy, comprising a CD4 detecting agent, a CCR4 detecting agent, and a CCR6 detecting agent, wherein the prediction is performed by using a relative amount of a CCR4⁻CCR6⁺ cell subpopulation in a CD4⁺ T cell population of the subject as an indicator for predicting a response of the subject to cancer therapy.

34. The kit of claim 33, wherein comparison of the relative amount with a reference value is used as an indicator for predicting a response of the subject to cancer therapy.

35. The kit of claim 33 or 34, wherein comparison of the relative amount with a reference value is used as an indicator for predicting long-term survival of the subject.

36. The kit of any one of claims 33 to 35, wherein comparison of the relative amount with a non-responder group threshold value is used as an indicator for predicting whether the subject is a part of a non-responder group to the cancer therapy.

37. The kit of any one of claims 33 to 36, wherein the detecting agent is an antibody.

38. The kit of any one of claims 33 to 37, further comprising a CXCR3 detecting agent.

39. The kit of any one of claims 33 to 38, wherein the cancer therapy comprises cancer immunotherapy, radiation therapy, molecularly targeted drug therapy, surgical operation, cell infusion, or any combination thereof.

40. The kit of any one of claims 33 to 39, wherein the cancer therapy is cancer immunotherapy.
